# EUROPEAN PATENT APPLICATION

(11) **EP 3 677 585 A1**
(43) Date of publication of application: **08.07.2020**
(21) Application number: 18850439.3
(22) Date of filing: 09.08.2018
(51) Int. Cl.: C07F 5/00, C23C 16/18, H01L 21/31

(54) **METAL ALKOXIDE COMPOUND, THIN-FILM-FORMING RAW MATERIAL, AND THIN FILM PRODUCTION METHOD**

(30) Priority: 30.08.2017 JP 2017165235
(71) Applicant: Adeka Corporation, Tokyo 116-8554 (JP)
(72) Inventor: OKADA, Nana, Tokyo 116-8554 (JP); HATASE, Masako, Tokyo 116-8554 (JP); NISHIDA, Akihiro, Tokyo 116-8554 (JP); SAKURAI, Atsushi, Tokyo 116-8554 (JP)
(74) Representative: Kador & Partner PartG mbB
(86) International application number: PCT/JP2018/029923
(87) International publication number: WO 2019/044448

(57) **Abstract**

The present invention provides a metal alkoxide compound represented by the following general formula (1), a thin-film-forming raw material containing the same, and a thin film production method of forming a metal-containing thin film using the raw material: 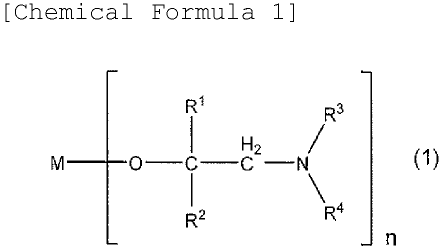 wherein R¹ represents a hydrogen atom or an alkyl group having 1 to 4 carbon atoms, R² represents an isopropyl group, a sec-butyl group, a tert-butyl group, a sec-pentyl group, a 1-ethylpropyl group or a tert-pentyl group, R³ represents a hydrogen atom or an alkyl group having 1 to 4 carbon atoms, R⁴ represents an alkyl group having 1 to 4 carbon atoms, M represents a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom or a lutetium atom, and n represents the valence of the atom represented by M; here, when M is a lanthanum atom, R² is a sec-butyl group, a tert-butyl group, a sec-pentyl group, a 1-ethylpropyl group or a tert-pentyl group.

## Description

### Technical Field

The present invention relates to a metal alkoxide compound having a specific amino alcohol as a ligand, a thin film forming raw material containing the compound, and a method of producing a metal-containing thin film using the thin-film-forming raw material.

### Background Art

In elements of Group 3 in the periodic table, scandium (Sc), yttrium (Y), and lanthanoids from lanthanum (La) to lutetium (Lu) are collectively referred to as rare earth elements, and such rare earth elements are important elements in the field of electronics and optronics. Among these, yttrium is a main constituent element of a Y-B-C-based superconductor. Lanthanum is a main constituent element of a ferroelectric PLZT. In addition, many lanthanoid elements are used as an additive for imparting functionality as a light-emitting material and the like.

A thin film containing such an element can be produced by a production method such as a sputtering method, an ion plating method, an MOD method such as a coating pyrolysis method and a sol-gel method, a chemical vapor deposition method, or the like. However, a chemical vapor deposition (hereinafter also simply referred to as "CVD") method including an atomic layer deposition (ALD) method is an optimal production process because it has many advantages such as excellent composition controllability and step coverage, being suitable for mass production, and enabling hybrid integration.

A large number of various raw materials have been reported as metal supply sources used for chemical vapor deposition methods. However, for example, in Patent Document 1, a cobalt tertiary amino alkoxide represented by Co[O-A-NR¹R²] (in the formula, A represents a linear or branched C₂ to C₁₀ alkylene group which may be substituted with a halogen atom, and R¹ and R² represent a linear or branched C₁ to C₇ alkyl group which may be substituted with a halogen atom) which can be used as thin film forming raw materials has been reported. In addition, in Non Patent Document 1, lutetium and yttrium alkoxide compounds which can be used as thin film forming raw materials are disclosed. However, there is low productivity with the alkoxide compounds disclosed in Non Patent Document 1 due to a low vapor pressure, and when these are used as thin film forming raw materials for an ALD method, there is a problem of a large amount of a residual carbon components being mixed into a thin film.

### Prior Art Documents

### Patent Document

[Patent Document 1] Korean Patent No.10-0675983

### Non Patent Document

[Non Patent Document 1] Inorg. Chem. 1997, 36, 3545-3552 "Volatile Donor-Functionalized Alkoxy Derivatives of Lutetium and Their Structural Characterization"

### Summary of the Invention

### Problems to be Solved by the Invention

When a raw material for chemical vapor deposition or the like is vaporized to form a metal-containing thin film on a surface of a substrate, a thin film forming material that can produce a metal-containing thin film having a high vapor pressure, a low melting point, and high quality is required. None of conventionally known thin film forming materials serving as rare earth element supply sources exhibits such physical properties. Regarding thin film forming materials serving as rare earth element supply sources, in order to improve productivity, a material having a high vapor pressure is urgently required.

Therefore, an object of the present invention is to provide a metal alkoxide compound having a high vapor pressure, a low melting point, and particularly suitable as thin-film-forming raw materials for a CVD method, thin-film-forming raw materials containing the compound, and a method of producing a metal-containing thin film using the thin-film-forming raw materials.

### Means for Solving the Problem

As a result of repeated examinations, the inventors found that a specific metal alkoxide compound can address the above problem and thus developed the present invention.

That is, the present invention provides a metal alkoxide compound represented by the following general formula (1), a thin-film-forming raw material containing the same, and a method of producing a thin film for forming a metal-containing thin film using the raw material. 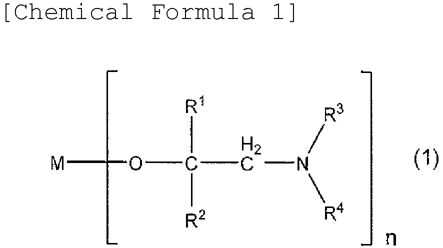 (In the formula, R¹ represents a hydrogen atom or an alkyl group having 1 to 4 carbon atoms, R² represents an isopropyl group, a sec-butyl group, a tert-butyl group, a sec-pentyl group, a 1-ethylpropyl group or a tert-pentyl group, R³ represents a hydrogen atom or an alkyl group having 1 to 4 carbon atoms, R⁴ represents an alkyl group having 1 to 4 carbon atoms, M represents a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom or a lutetium atom, and n represents the valence of the atom represented by M; here, when M is a lanthanum atom, R² is a sec-butyl group, a tert-butyl group, a sec-pentyl group, a 1-ethylpropyl group or a tert-pentyl group)

### Effects of the Invention

According to the present invention, it is possible to obtain a metal alkoxide compound having a high vapor pressure, and the compound is suitable as a thin film forming raw material for a CVD method, and most especially, can be preferably used as a thin film forming raw material for an ALD method.

### Brief Description of Drawings

Fig. 1 is a schematic view showing an example of a chemical vapor deposition device used in a method of producing a thin film according to the present invention.
Fig. 2 is a schematic view showing another example of the chemical vapor deposition device used in a method of producing a thin film according to the present invention.
Fig. 3 is a schematic view showing still another example of the chemical vapor deposition device used in a method of producing a thin film according to the present invention.
Fig. 4 is a schematic view showing yet another example of the chemical vapor deposition device used in a method of producing a thin film according to the present invention.

### Best Mode for Carrying Out the Invention

A metal alkoxide compound of the present invention is represented by general formula (1) and is suitable as a precursor in a thin film production method including a vaporization process such as a CVD method and is a precursor that can be applied for an ALD method, and thus is particularly suitable as a precursor used in the ALD method.

In general formula (1), R¹ represents a hydrogen atom or an alkyl group having 1 to 4 carbon atoms, R² represents an isopropyl group, a sec-butyl group, a tert-butyl group, a sec-pentyl group, a 1-ethylpropyl group or a tert-pentyl group, R³ represents a hydrogen atom or an alkyl group having 1 to 4 carbon atoms, R⁴ represents an alkyl group having 1 to 4 carbon atoms, M represents a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom or a lutetium atom, and n represents the valence of the atom represented by M. Here, when M is a lanthanum atom, R² is a sec-butyl group, a tert-butyl group, a sec-pentyl group, a 1-ethylpropyl group or a tert-pentyl group.

In general formula (1), examples of alkyl groups having 1 to 4 carbon atoms represented by R¹, R³ and R⁴ include methyl, ethyl, propyl, isopropyl, butyl, isobutyl, sec-butyl, and tert-butyl groups.

n represents the valence of the atom represented by M. It is known that metal atoms represented by M in general formula (1) have various valences, and among them, regarding a stable valence, for example, a case in which n is 3 when M is a scandium, yttrium, lanthanum, promethium, samarium, gadolinium, dysprosium, holmium, erbium, or lutetium atom, a case in which n is 3 or 4 when M is a cerium, praseodymium, or terbium atom, and a case in which n is 2 or 3 when M is a neodymium, europium, thulium, or ytterbium atom are known.

The metal alkoxide compound represented by general formula (1) may have optical activity, but the metal alkoxide compound of the present invention may be any of an R form and an S form without particular distinction or a mixture containing an R form and an S form in a certain ratio. The racemate is inexpensive to produce.

When the metal alkoxide compound represented by general formula (1) is used in a method of producing a thin film including a process of vaporizing a metal alkoxide compound, a metal alkoxide compound having a high vapor pressure is preferable. Therefore, regarding R¹ to R³ in general formula (1), specifically, those in which R¹ is a hydrogen atom and R² is a tert-butyl or tert-pentyl group are preferable because they have a high vapor pressure. Among these, those in which R¹ is a hydrogen atom, and R² is a tert-butyl group are preferable, and those in which R¹ is a hydrogen atom, R² is a tert-butyl group, R³ is a hydrogen atom or a methyl or ethyl group, and R⁴ is a methyl or ethyl group are preferable because they have a particularly high vapor pressure. In addition, in a method of producing a thin film according to an MOD method not including a vaporization process of a metal alkoxide compound, R¹ to R³ can be arbitrarily selected according to the solubility in a solvent to be used, a thin film formation reaction, and the like.

While the metal alkoxide compound of the present invention is represented by general formula (1), it is a concept including a case in which a ring structure in which a terminal donor group in the ligand is coordinated to a metal atom is formed, that is, also a case in which it is represented by the following general formula (1-A) without distinction. 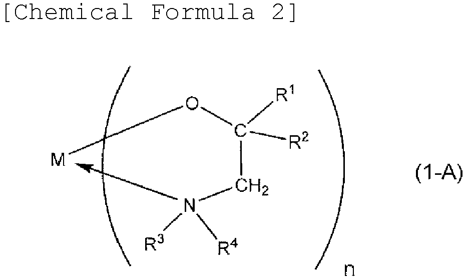 (In the formula, R¹ represents a hydrogen atom or an alkyl group having 1 to 4 carbon atoms, R² represents an isopropyl group, a sec-butyl group, a tert-butyl group, a sec-pentyl group, a 1-ethylpropyl group or a tert-pentyl group, R³ represents a hydrogen atom or an alkyl group having 1 to 4 carbon atoms, R⁴ represents an alkyl group having 1 to 4 carbon atoms, M represents a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom or a lutetium atom, and n represents the valence of the atom represented by M; here, when M is a lanthanum atom, R² is a sec-butyl group, a tert-butyl group, a sec-pentyl group, a 1-ethylpropyl group or a tert-pentyl group)

Specific examples of the metal alkoxide compound represented by general formula (1) include Compound No. 1 to Compound No. 196. Here, "Me" in the following chemical formulas represents a methyl group, "Et" represents an ethyl group, "iPr" represents an isopropyl group, and "tBu" represents a tert-butyl group.

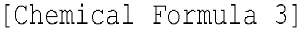

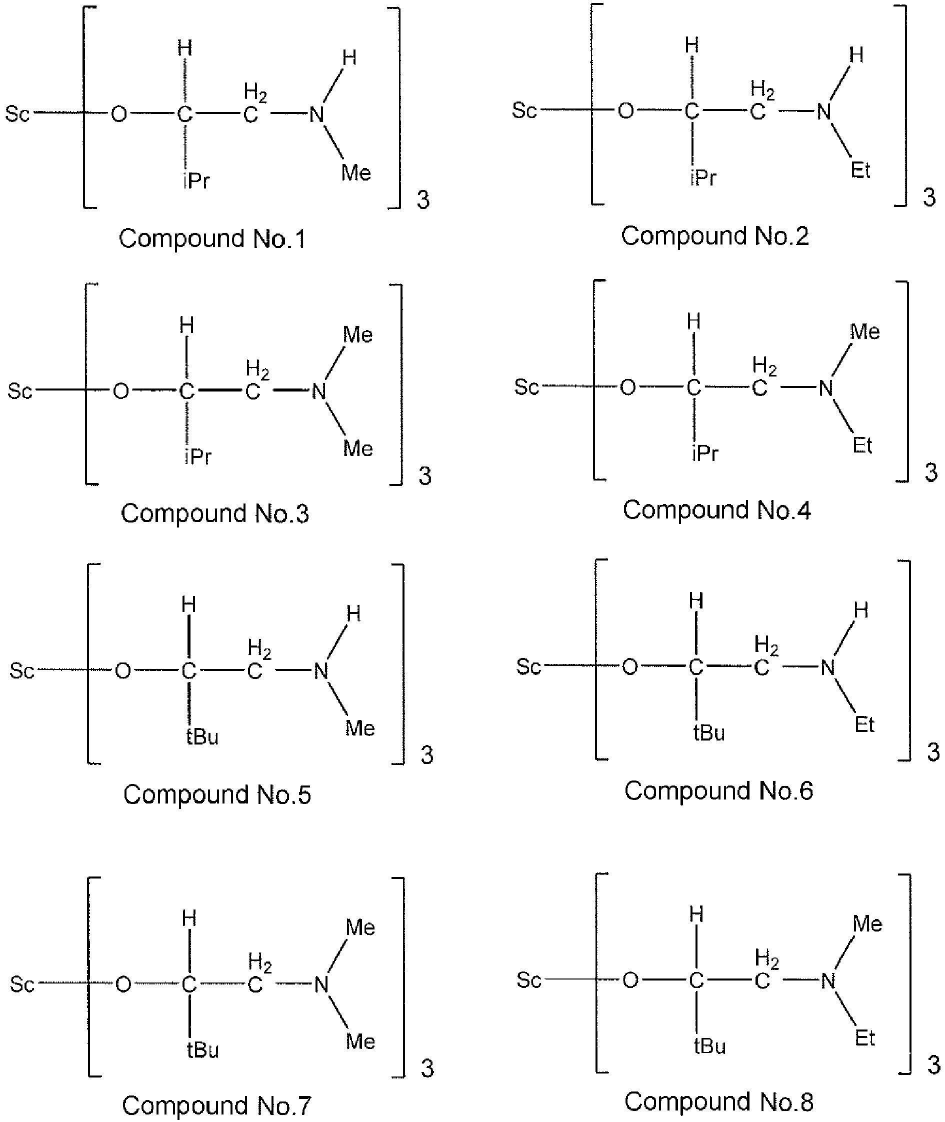

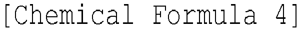

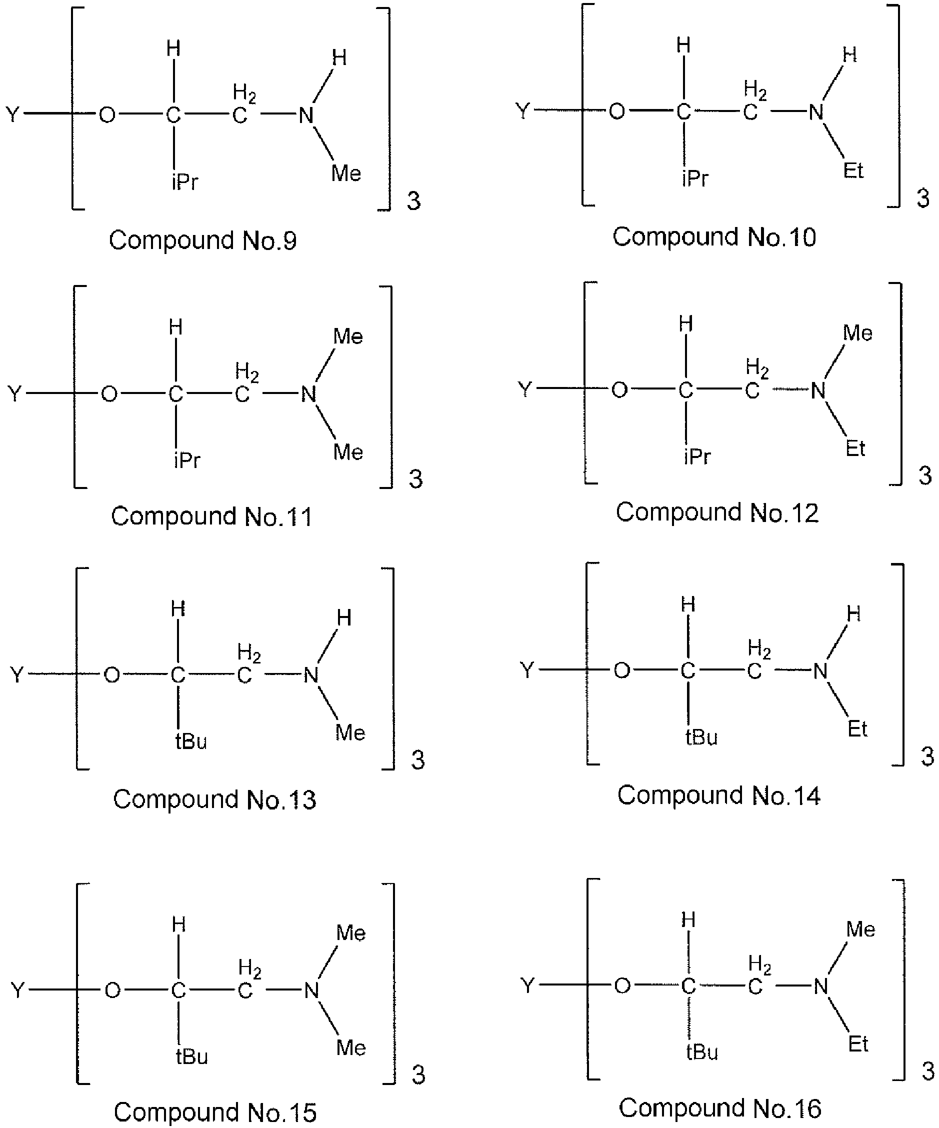

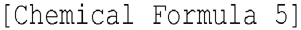

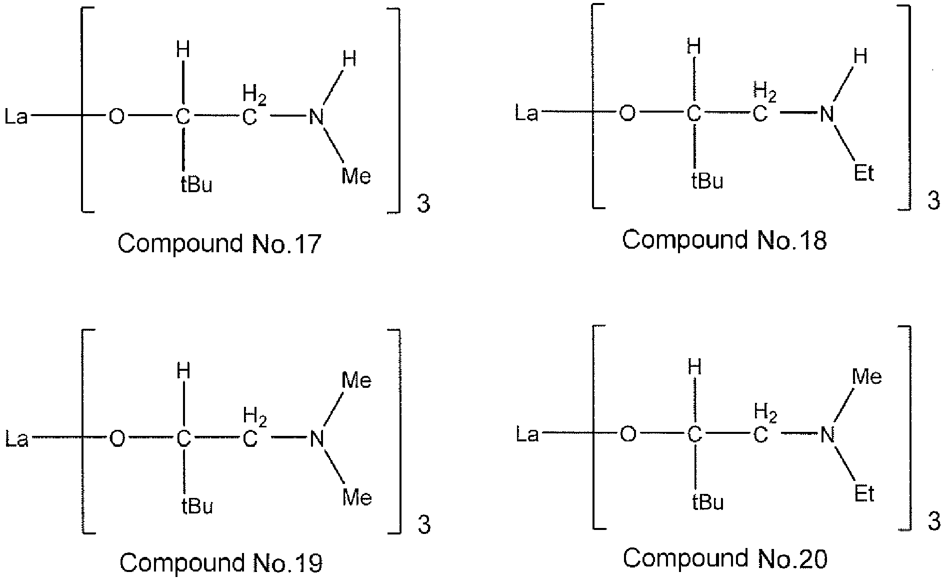

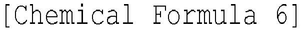

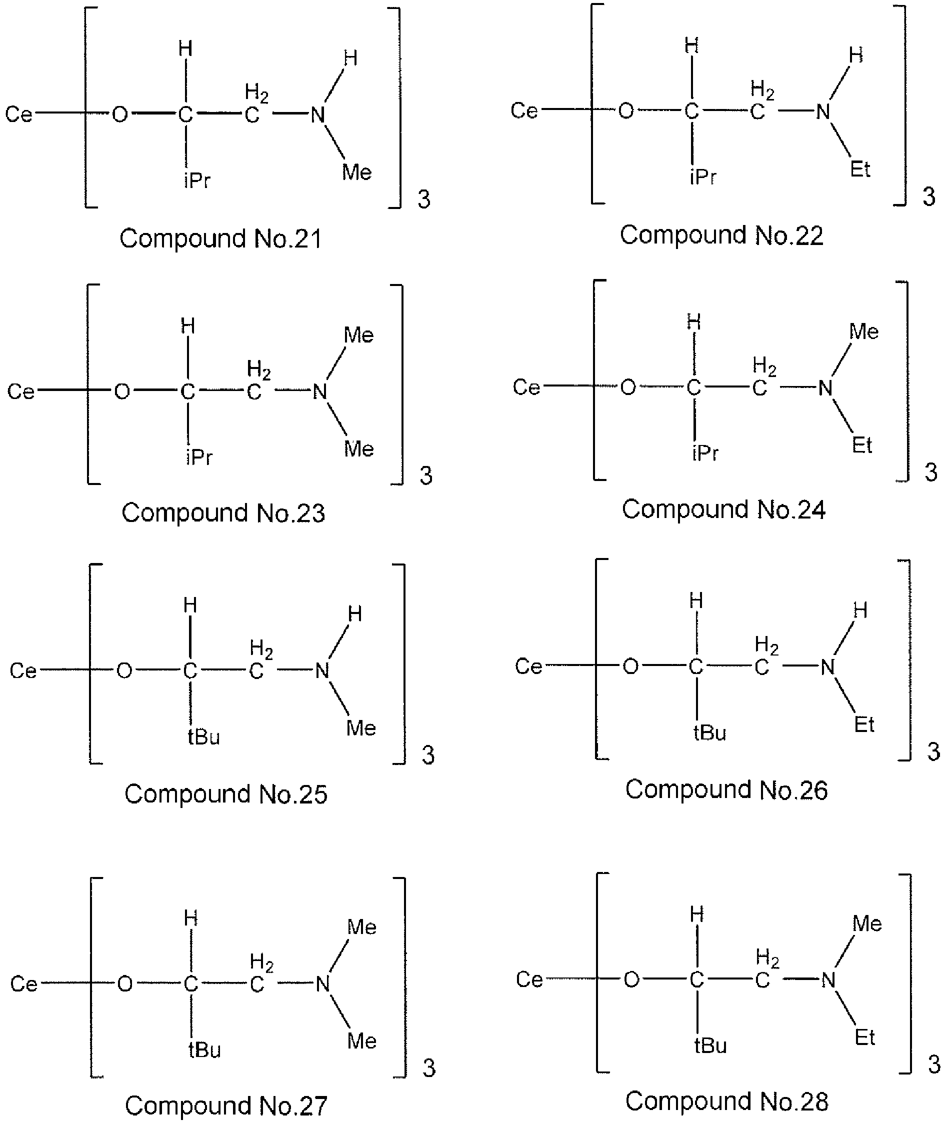

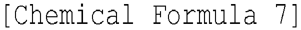

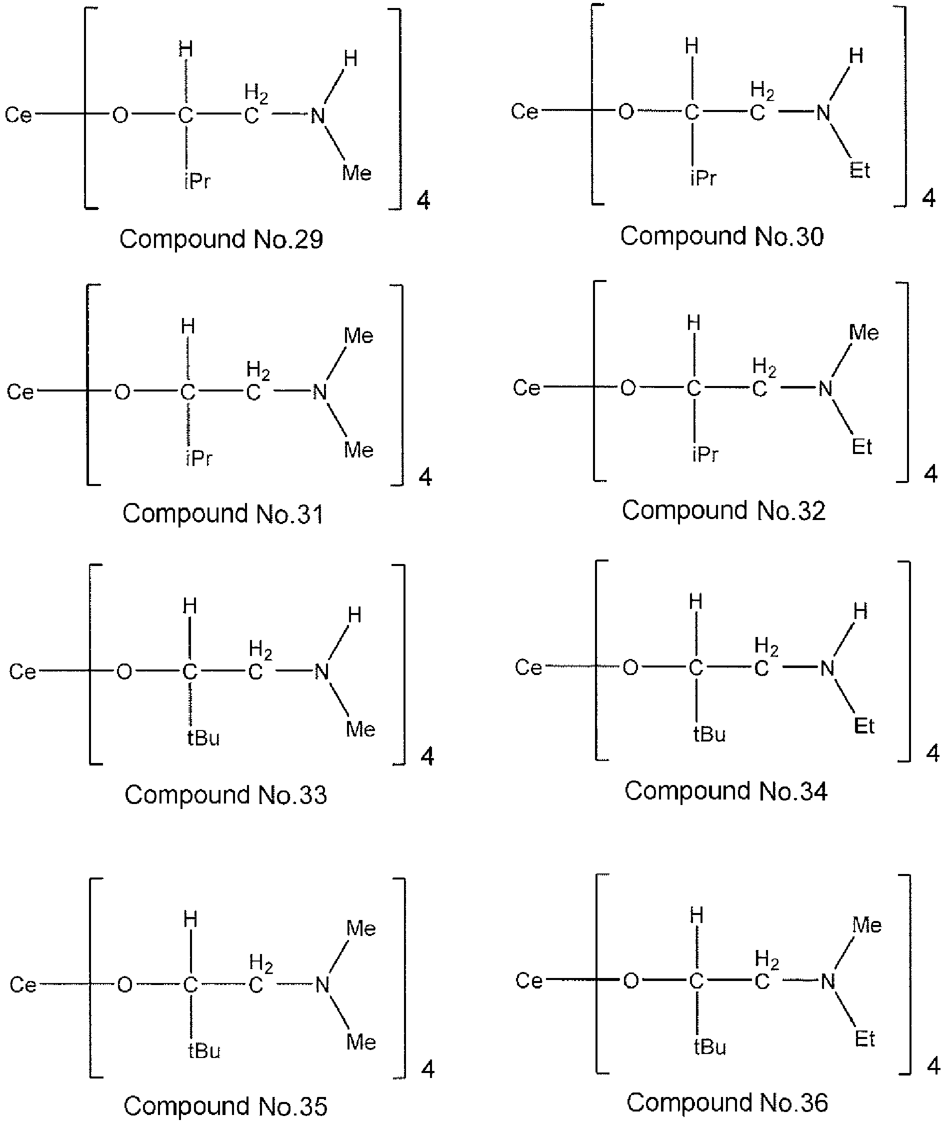

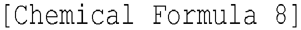

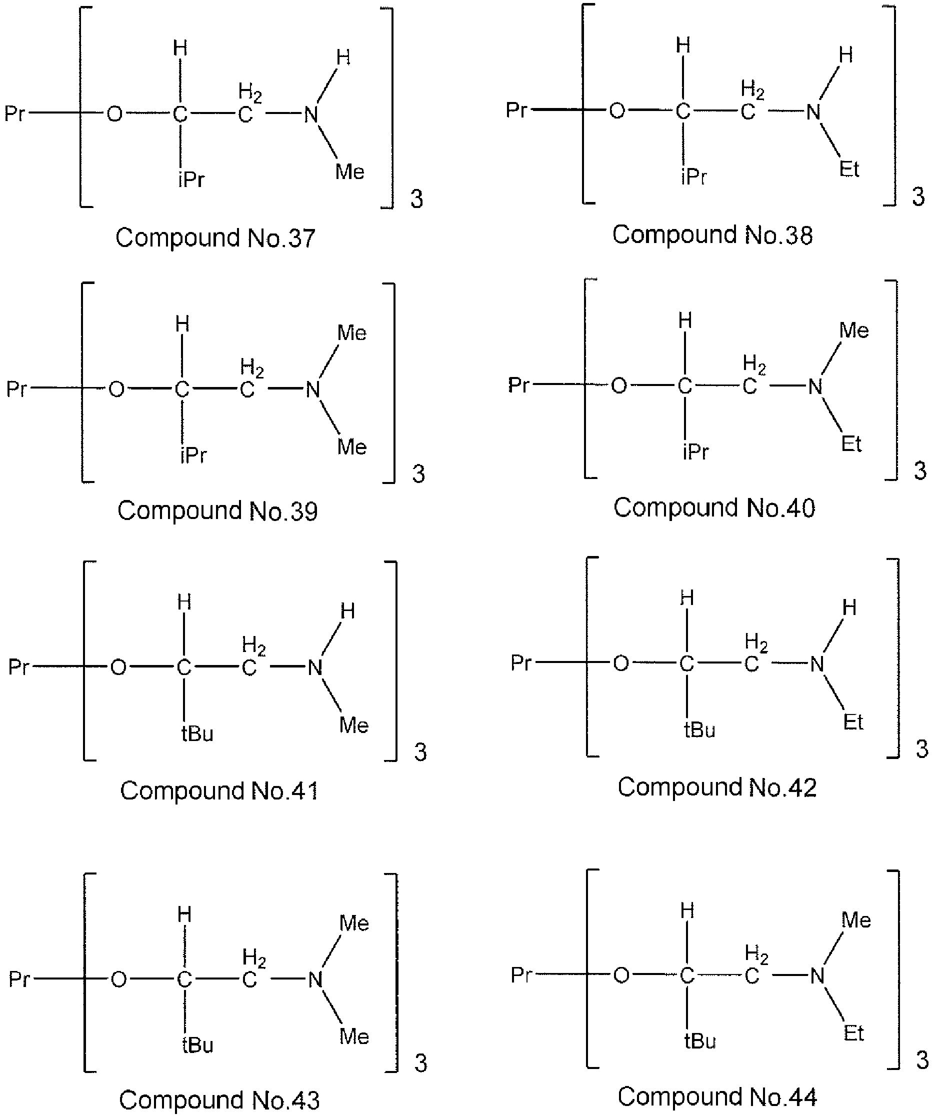

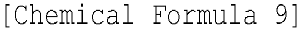

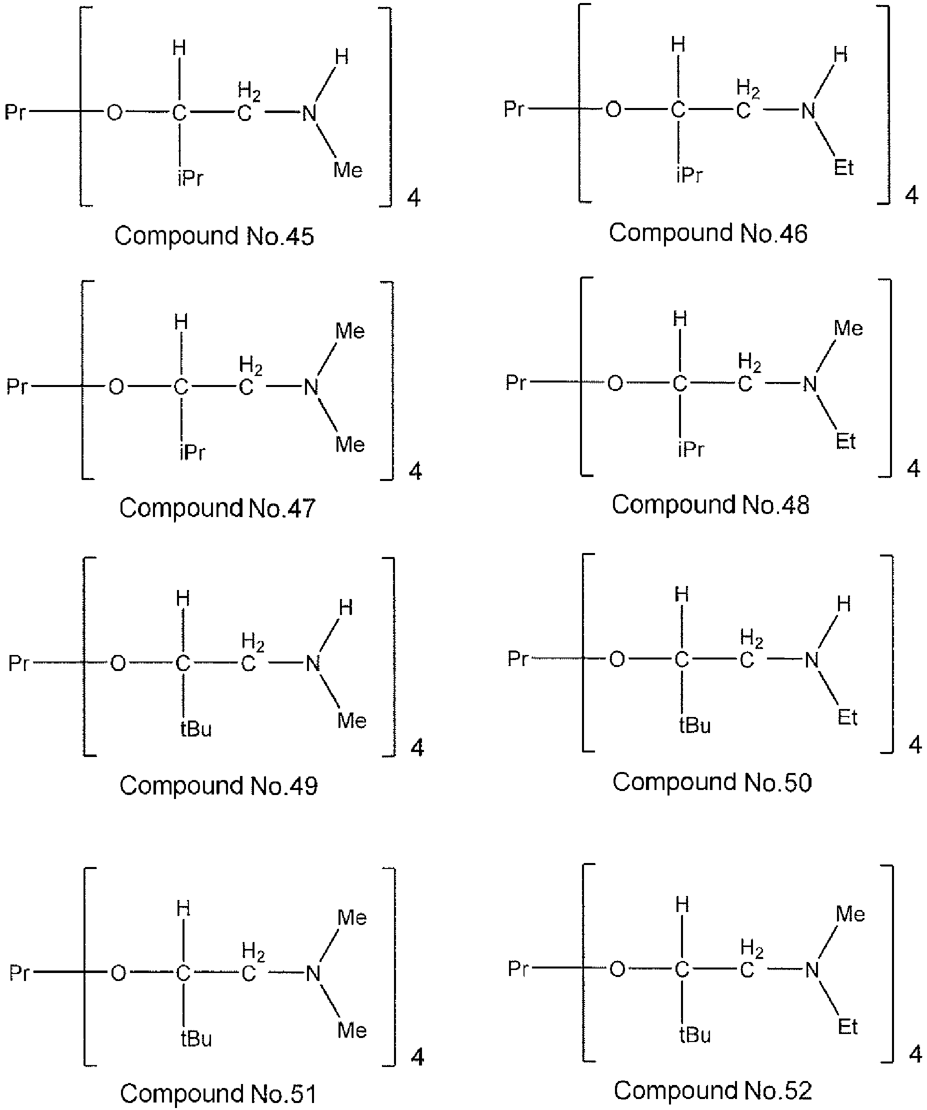

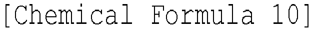

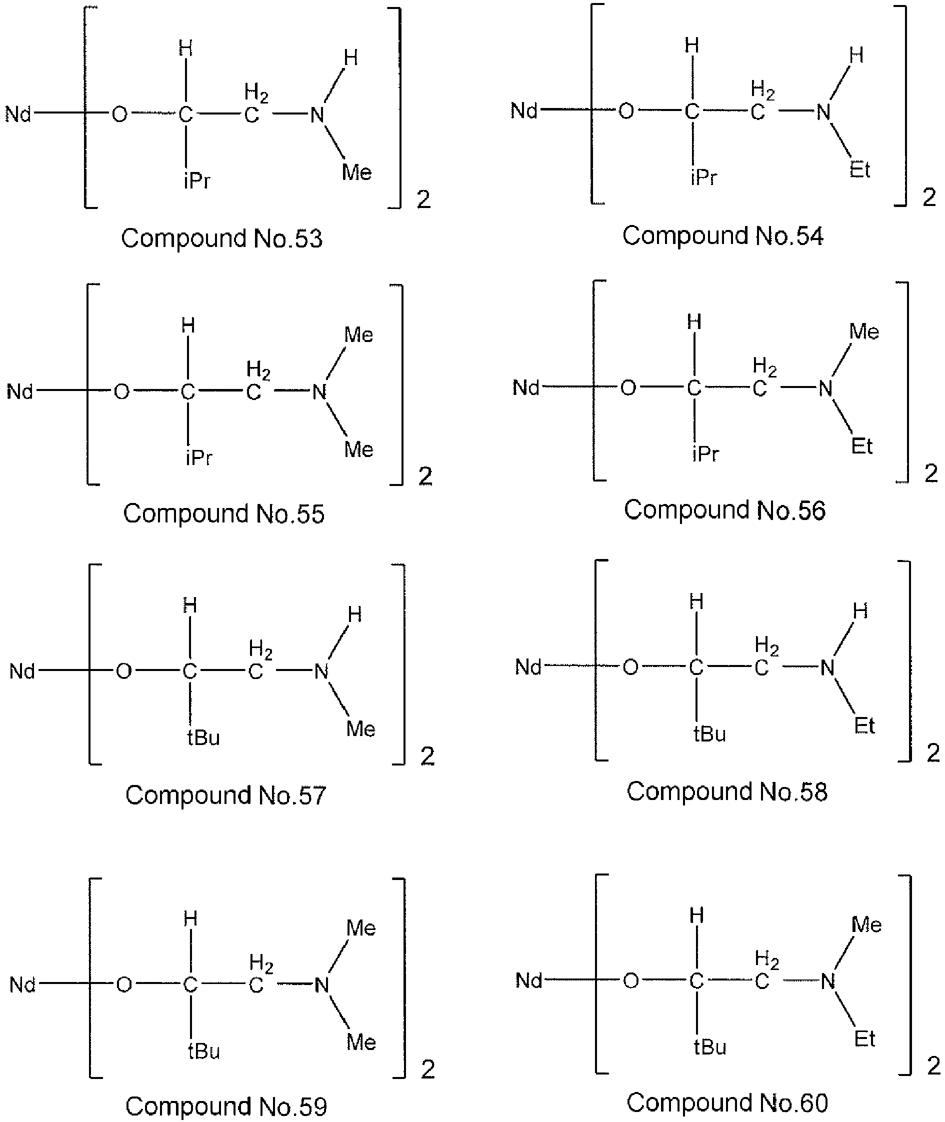

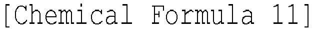

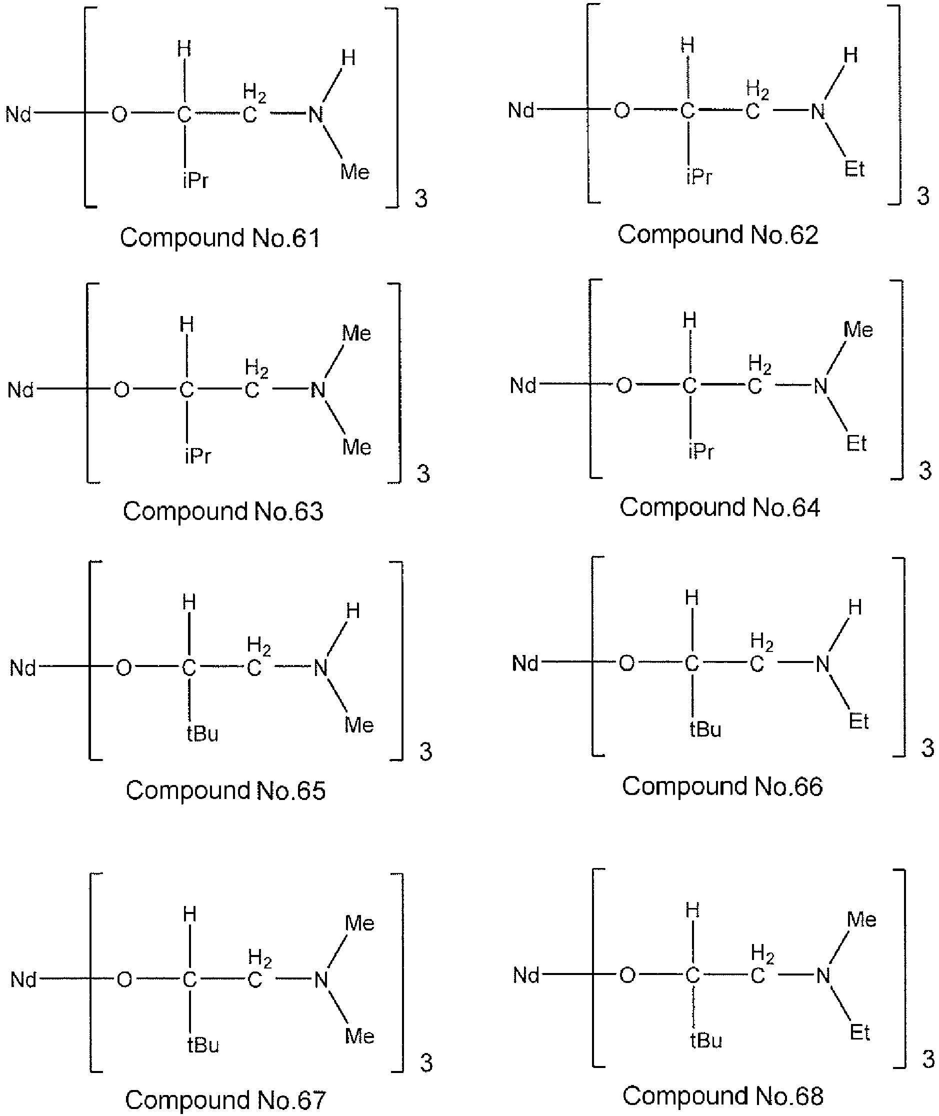

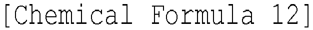

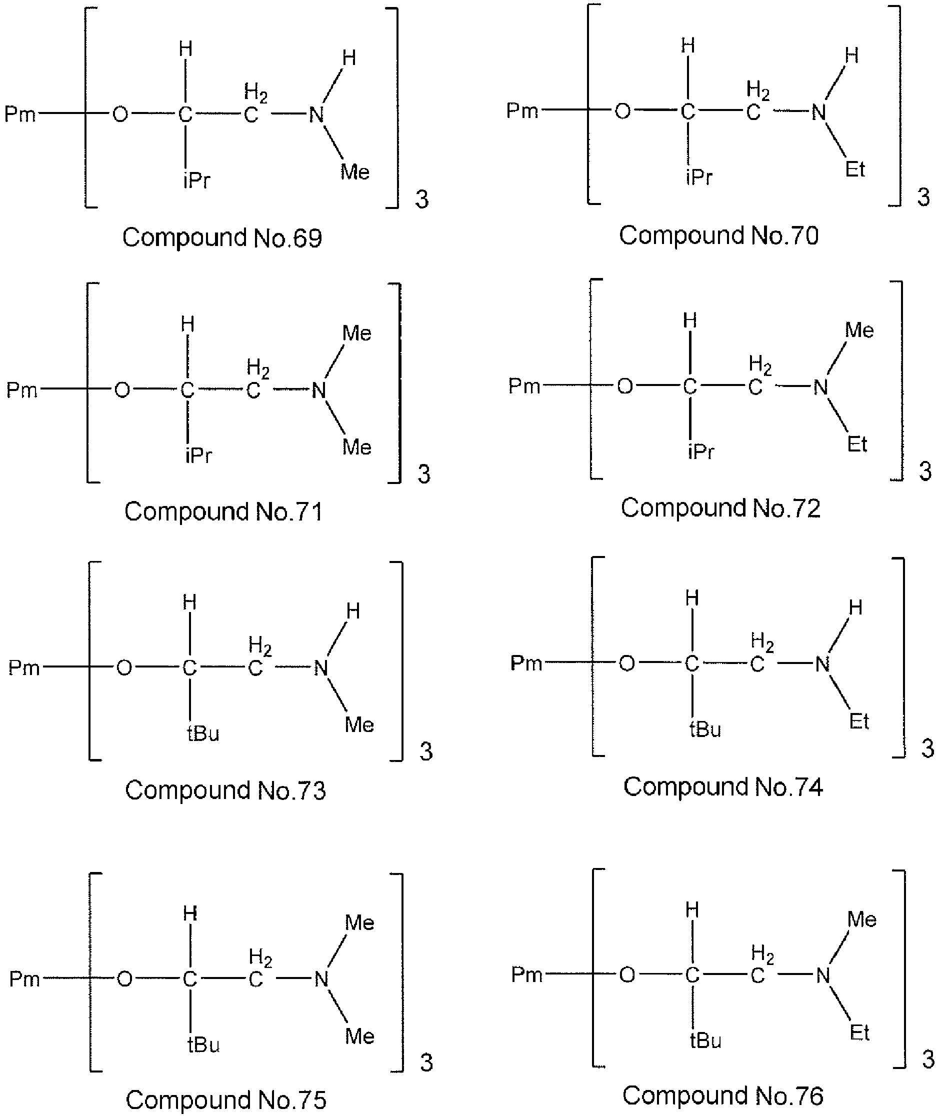

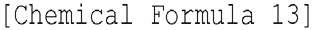

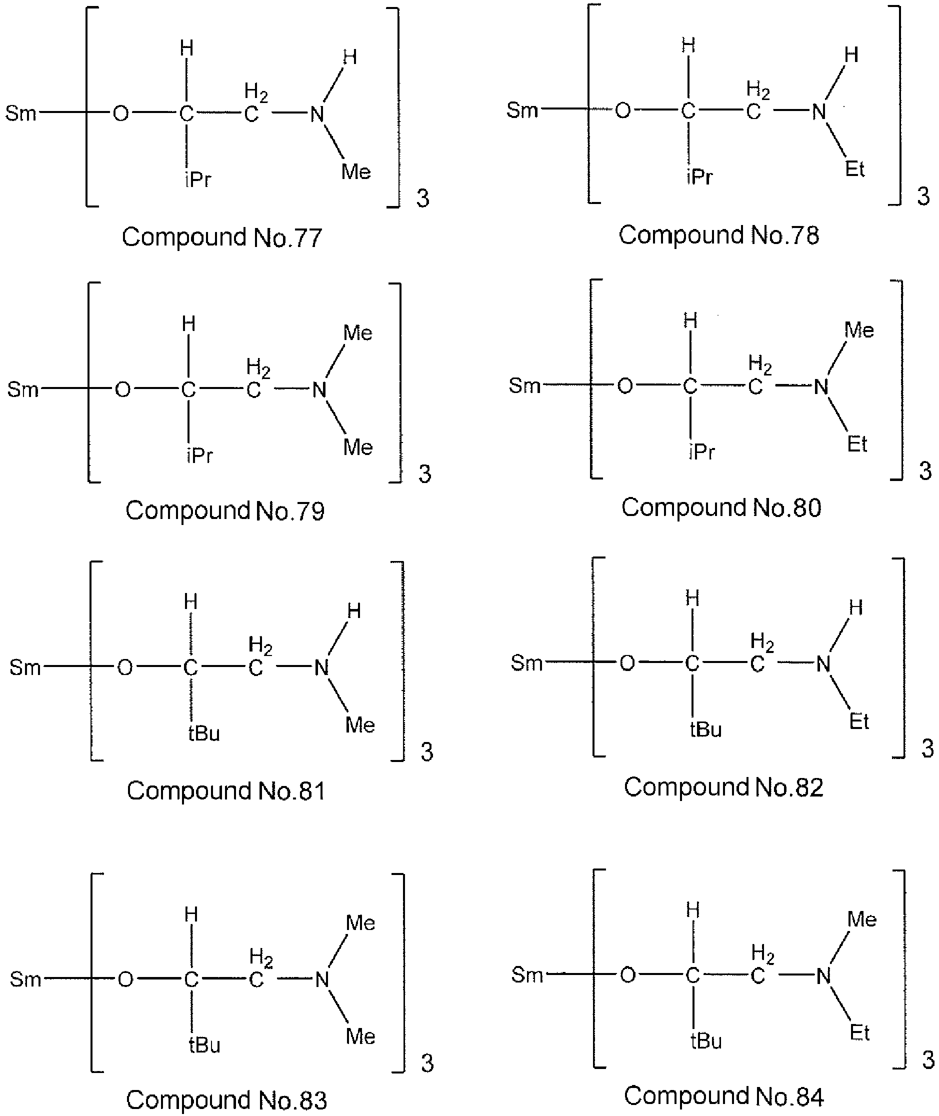

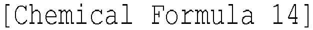

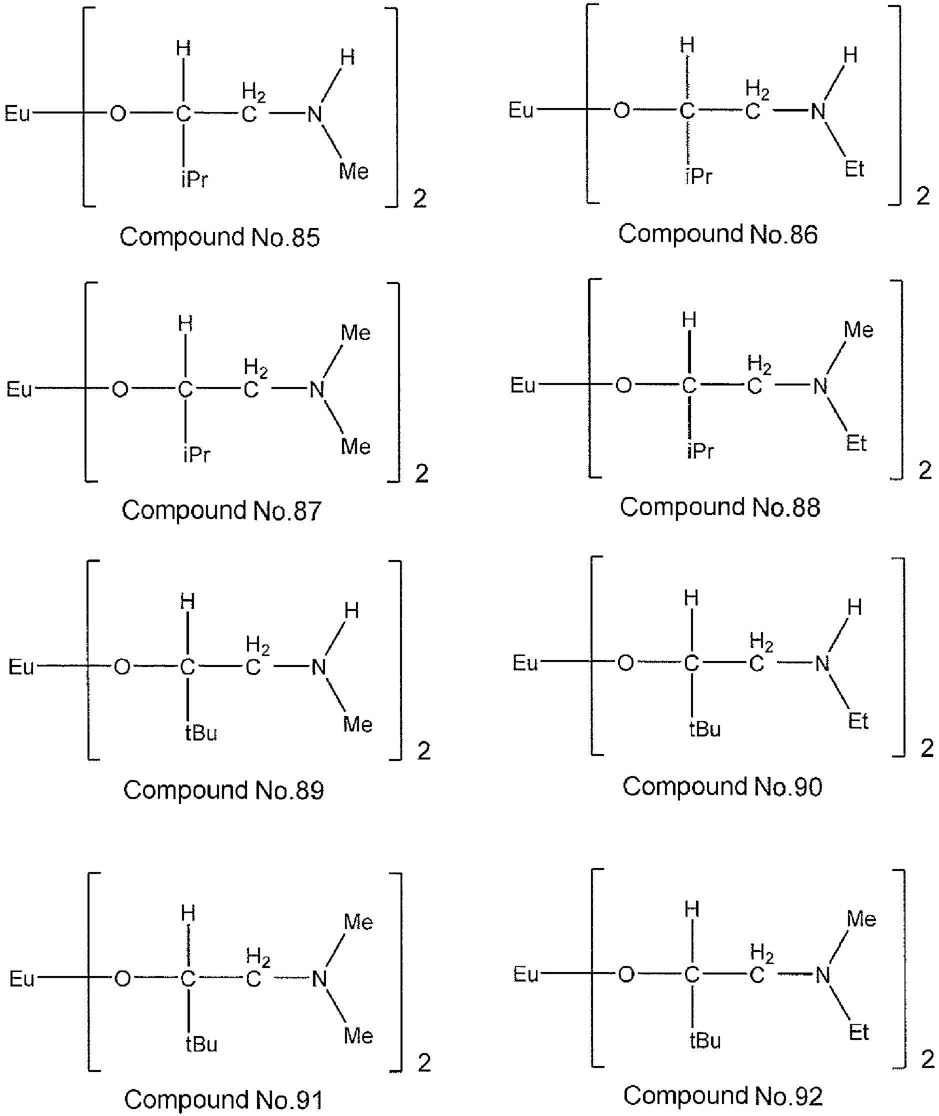

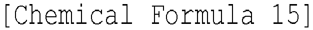

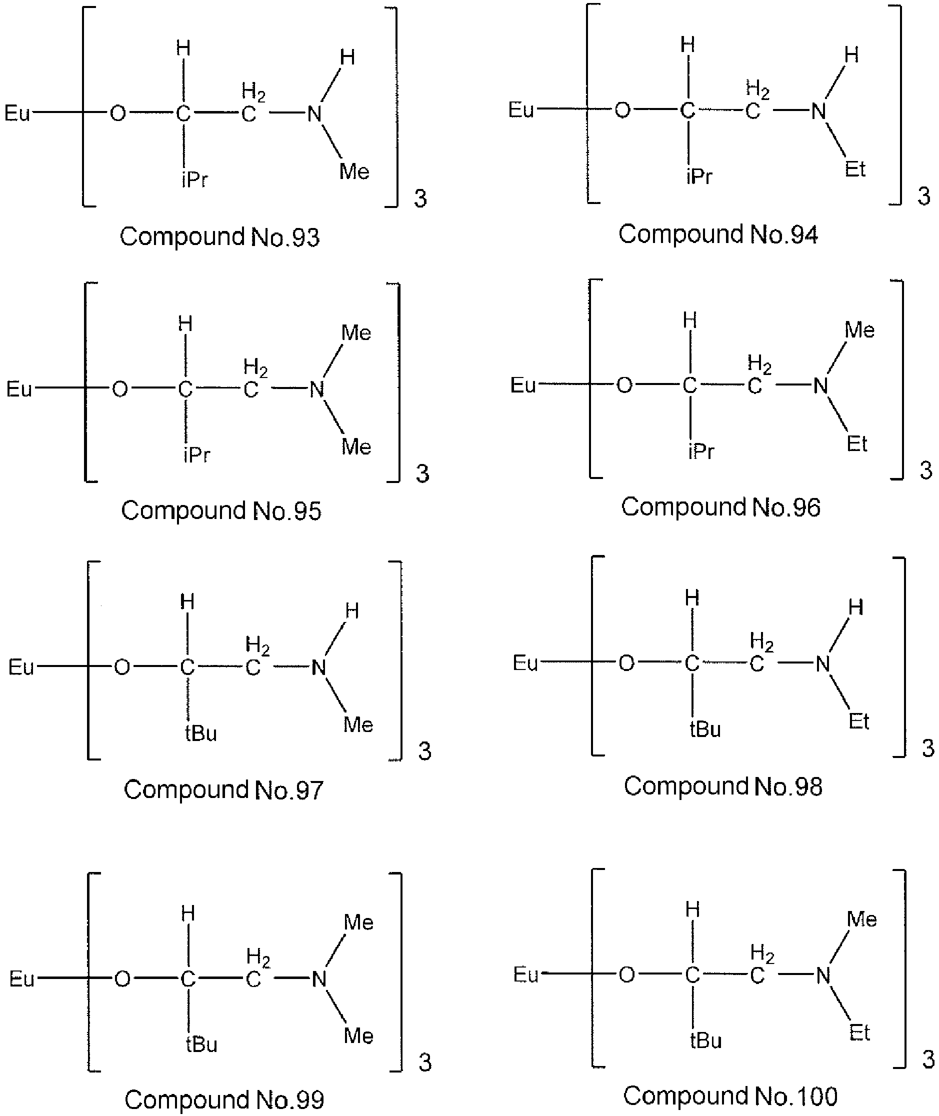

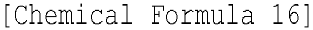

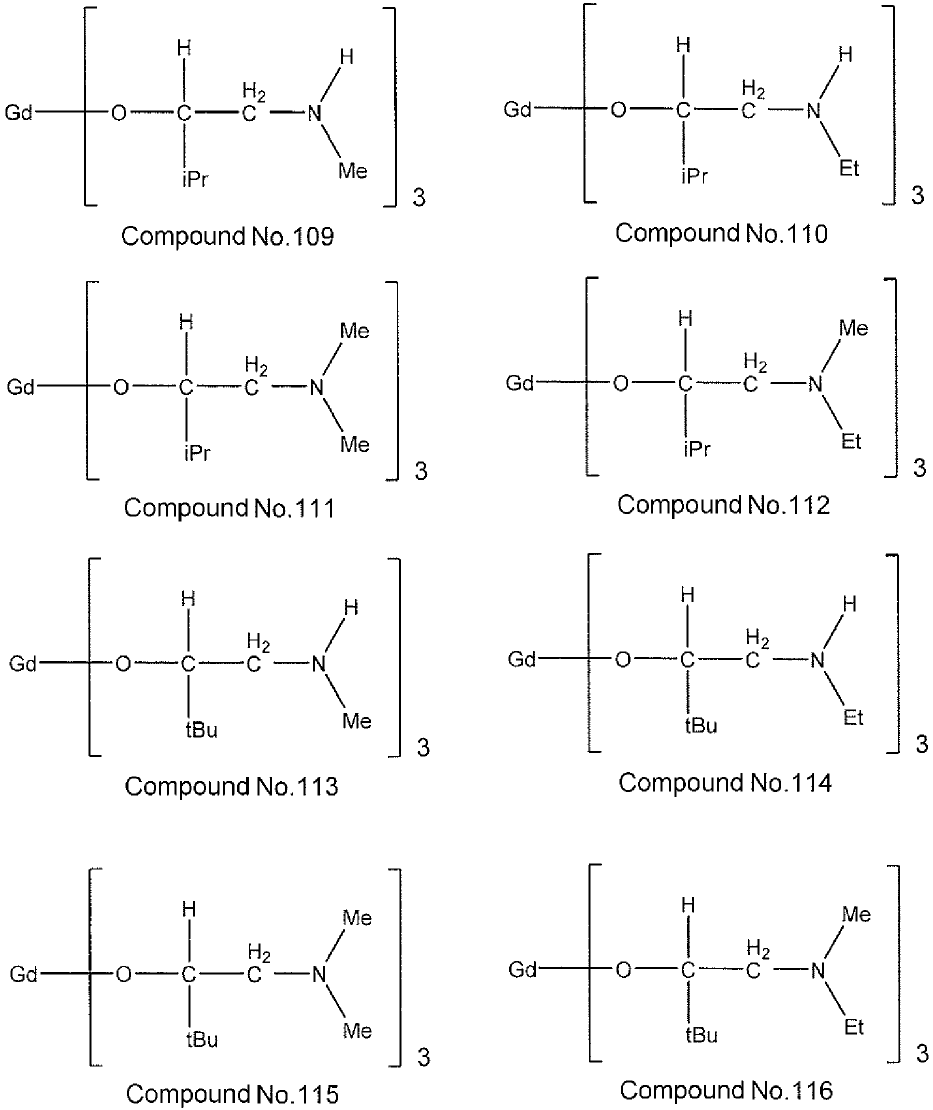

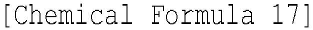

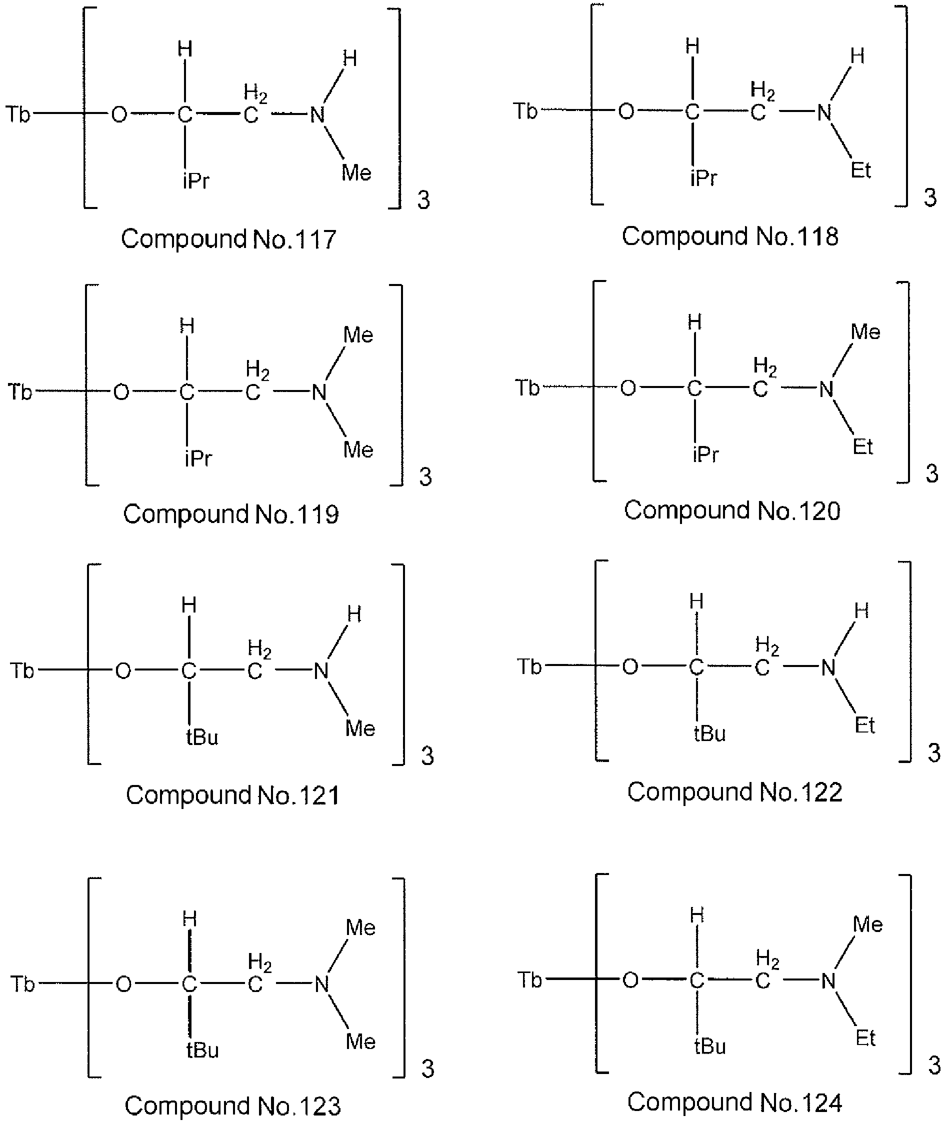

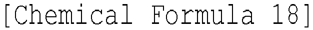

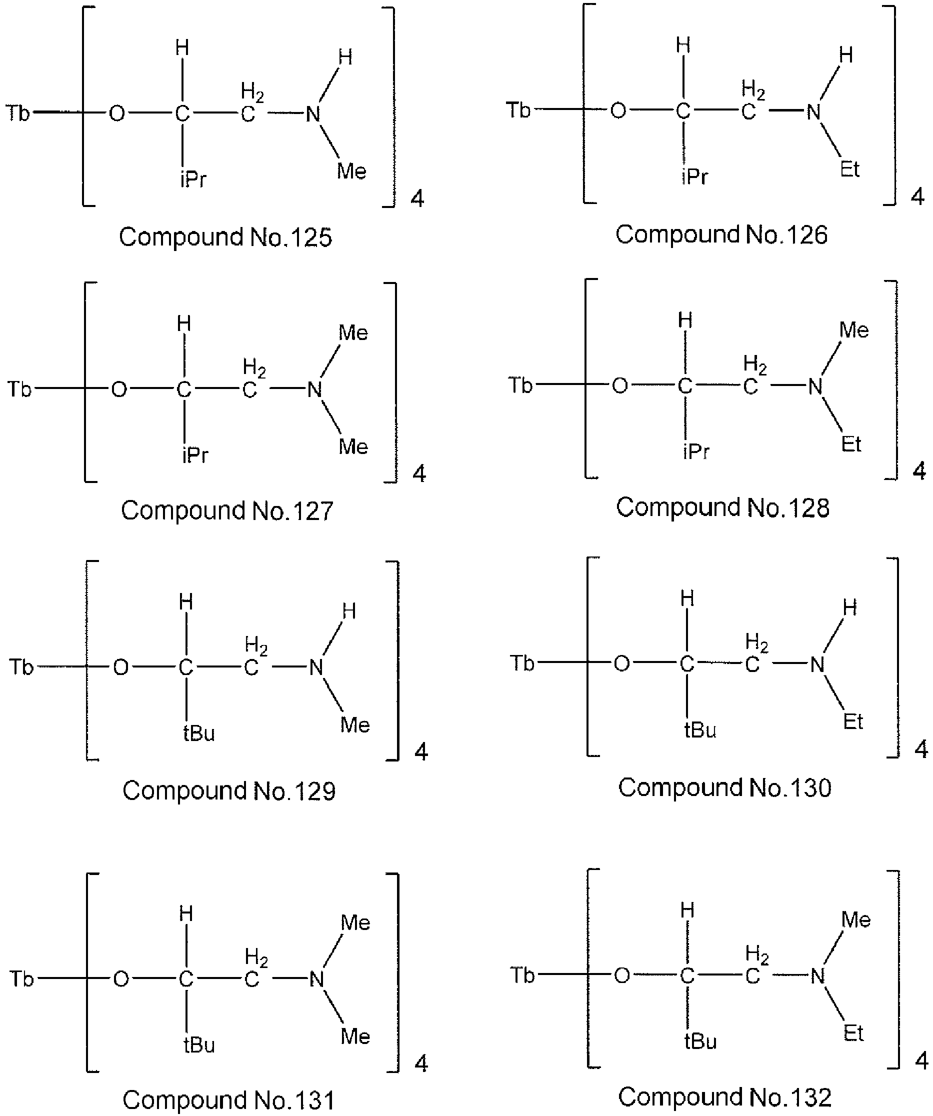

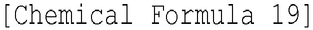

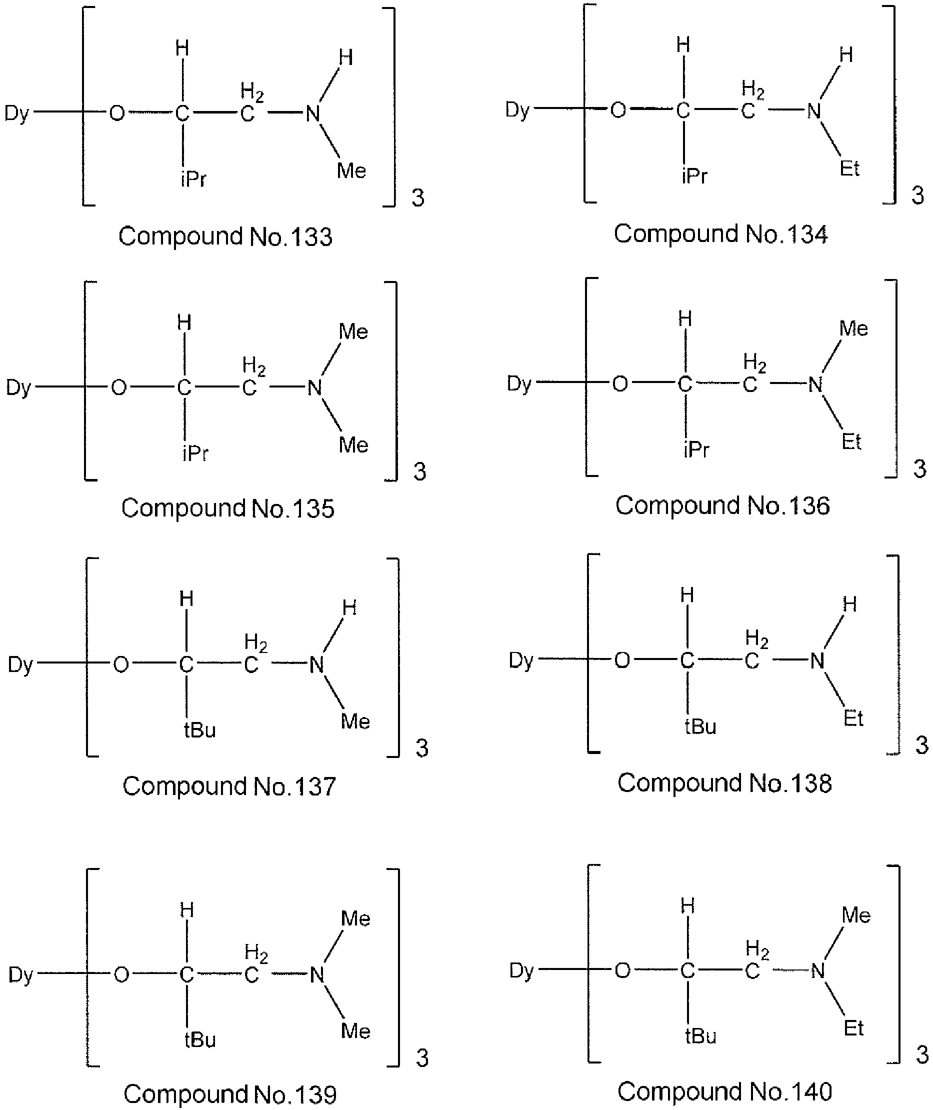

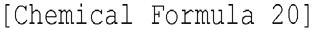

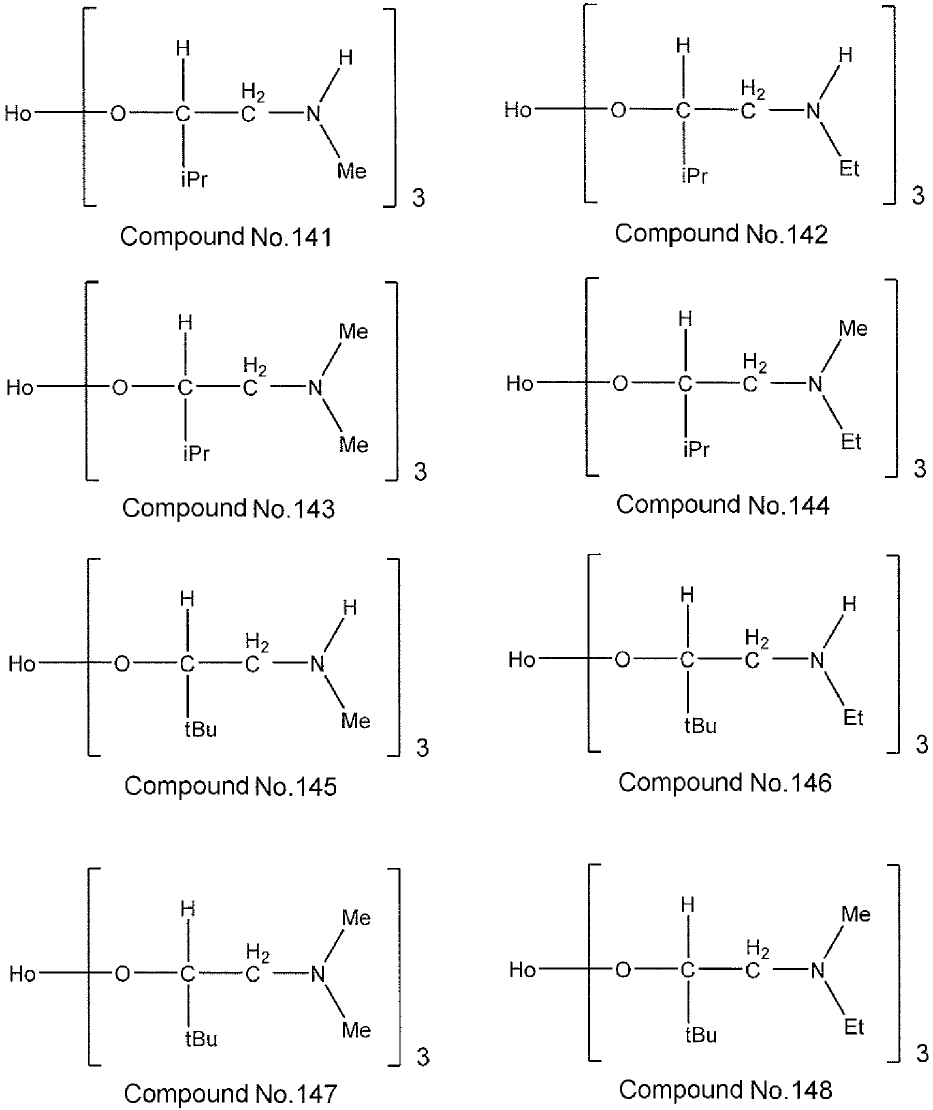

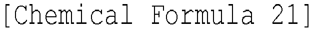

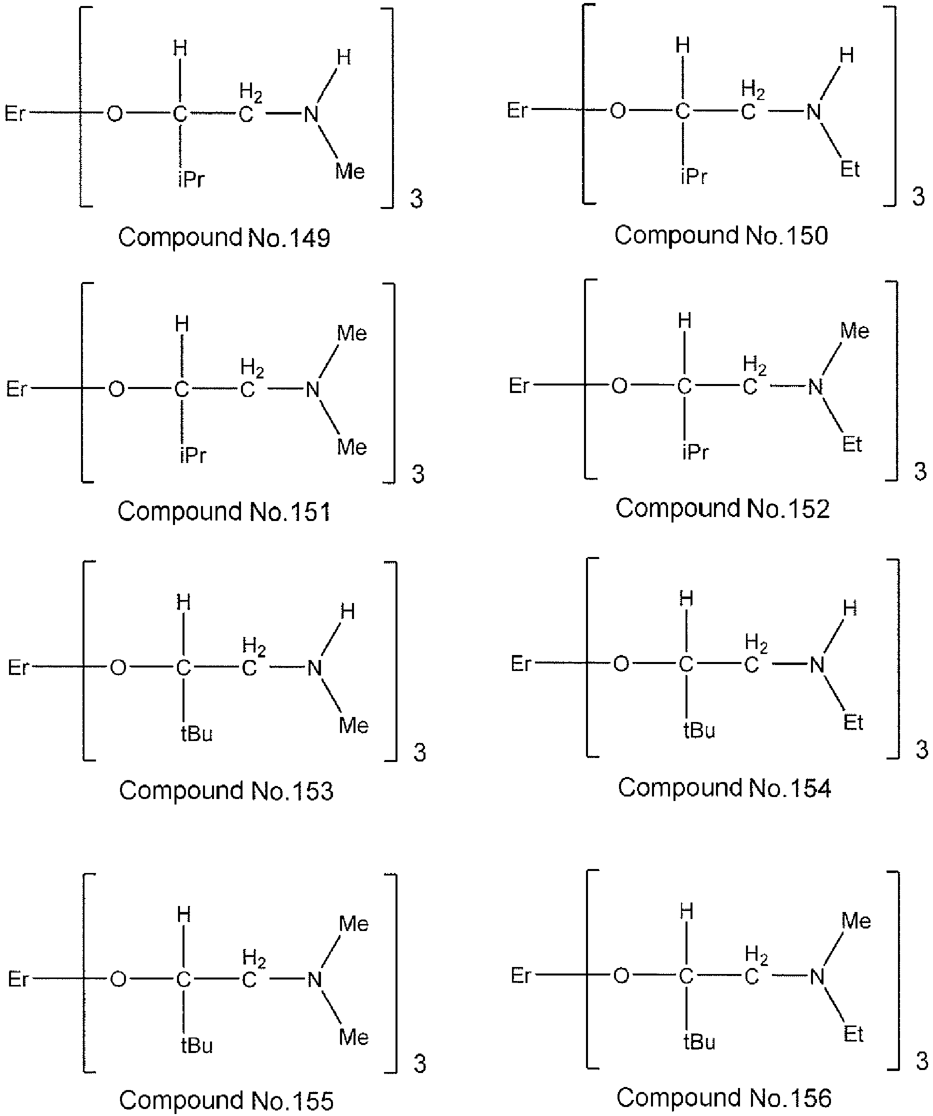

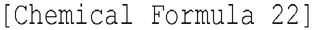

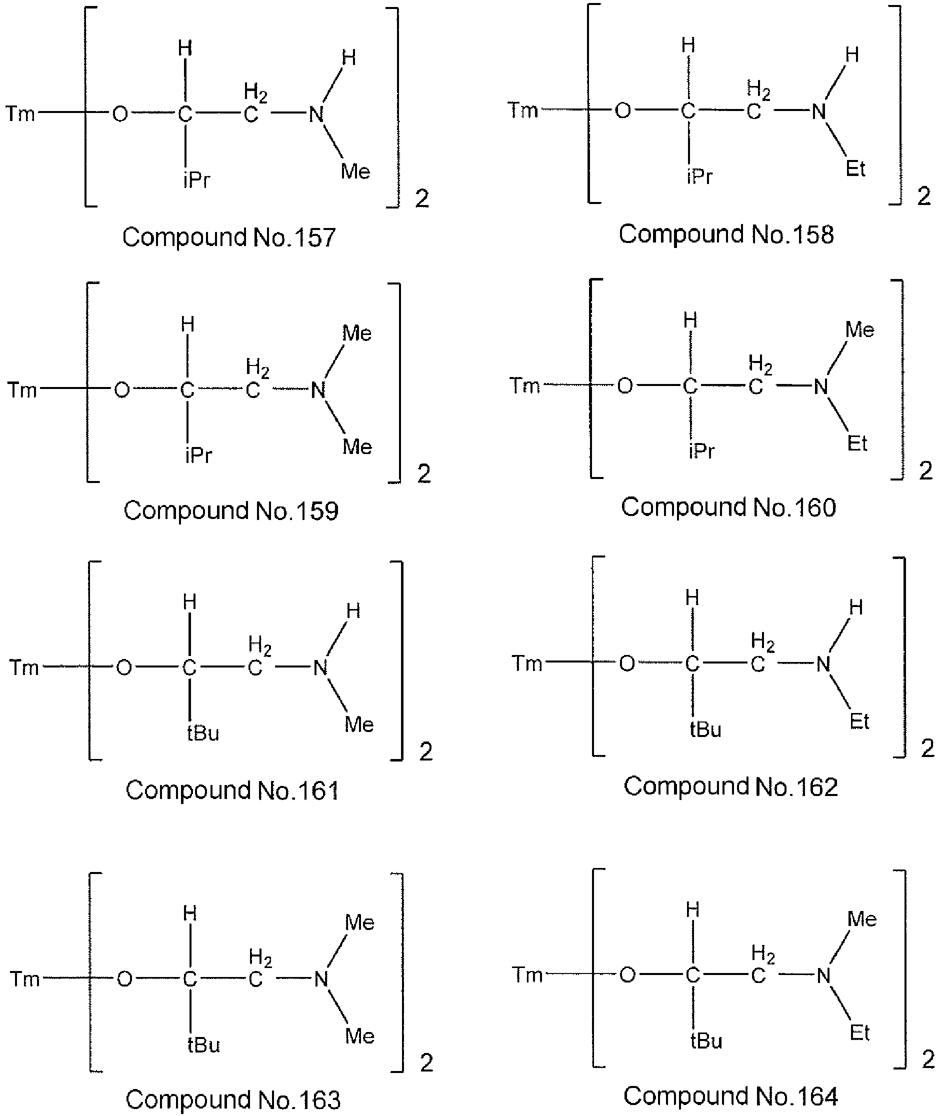

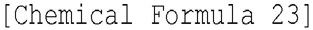

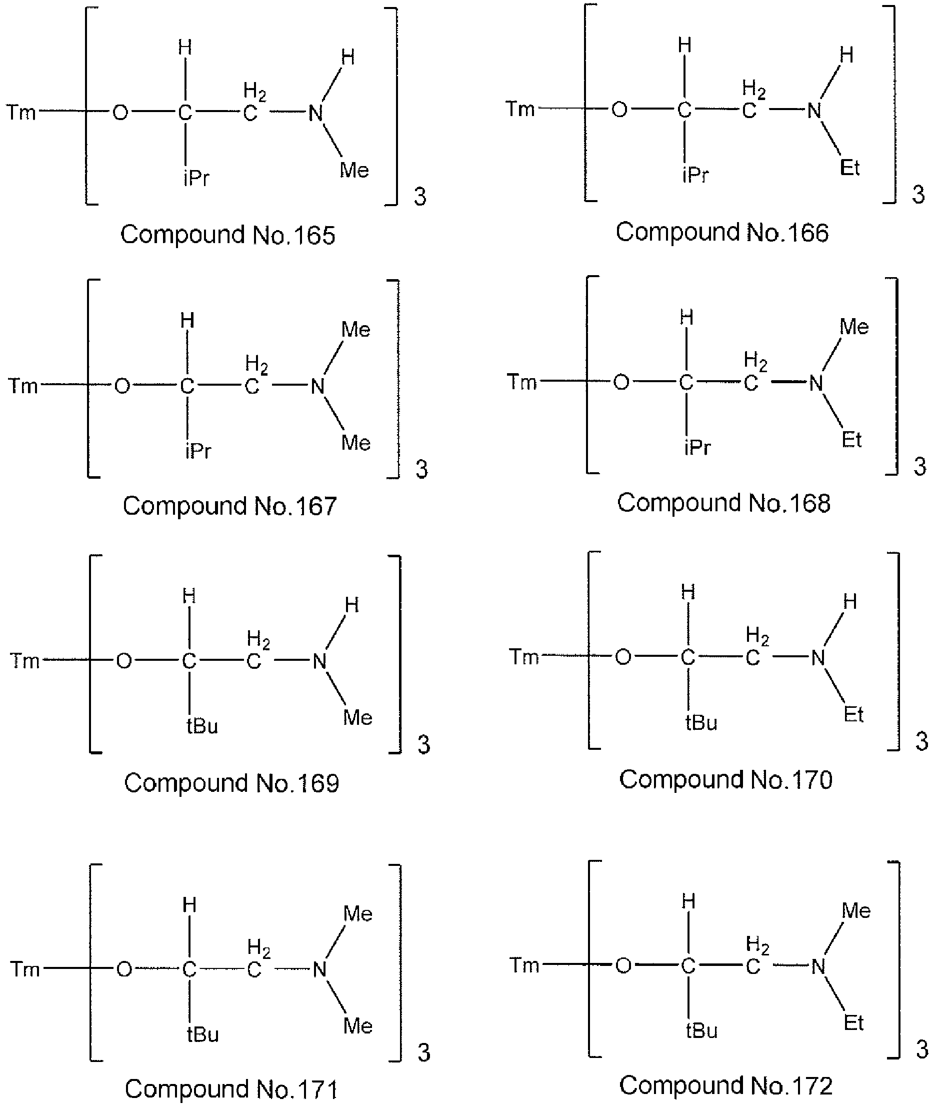

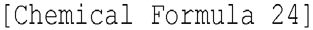

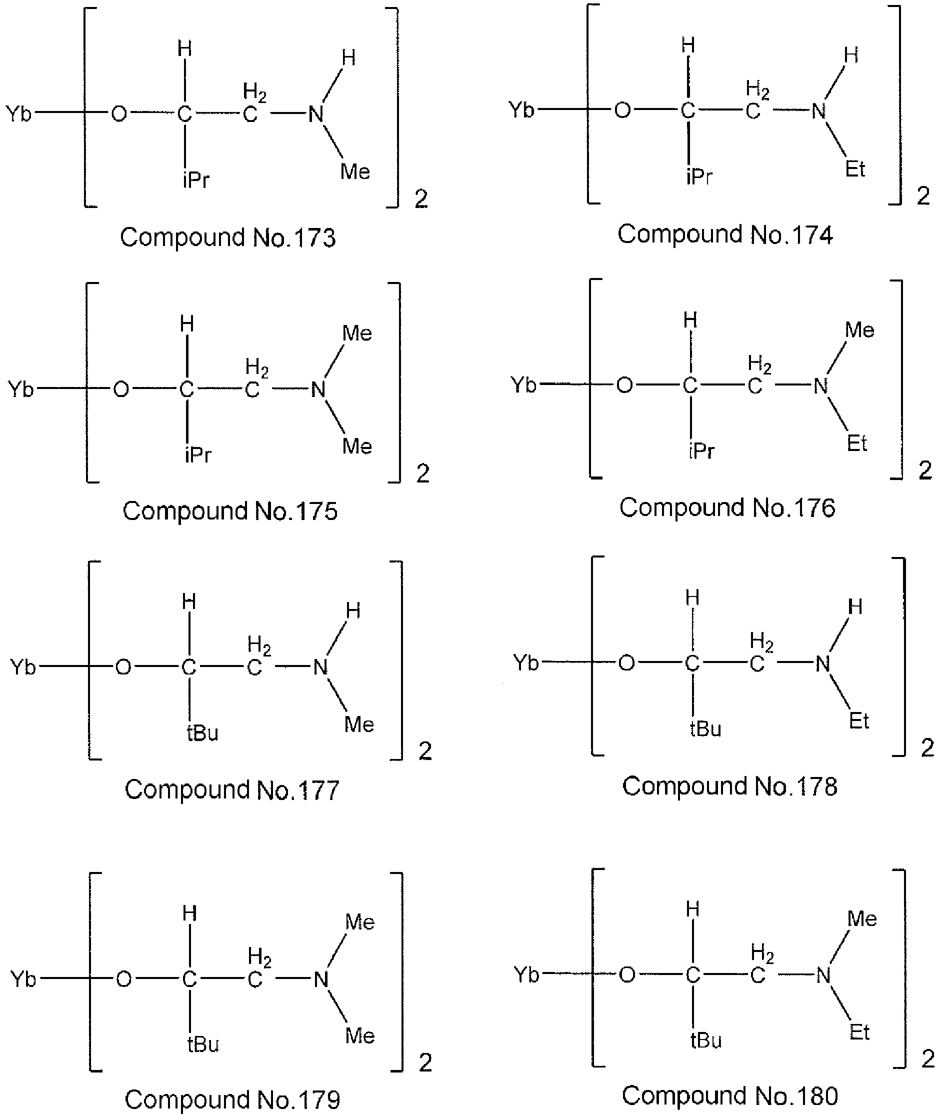

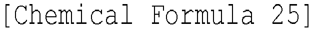

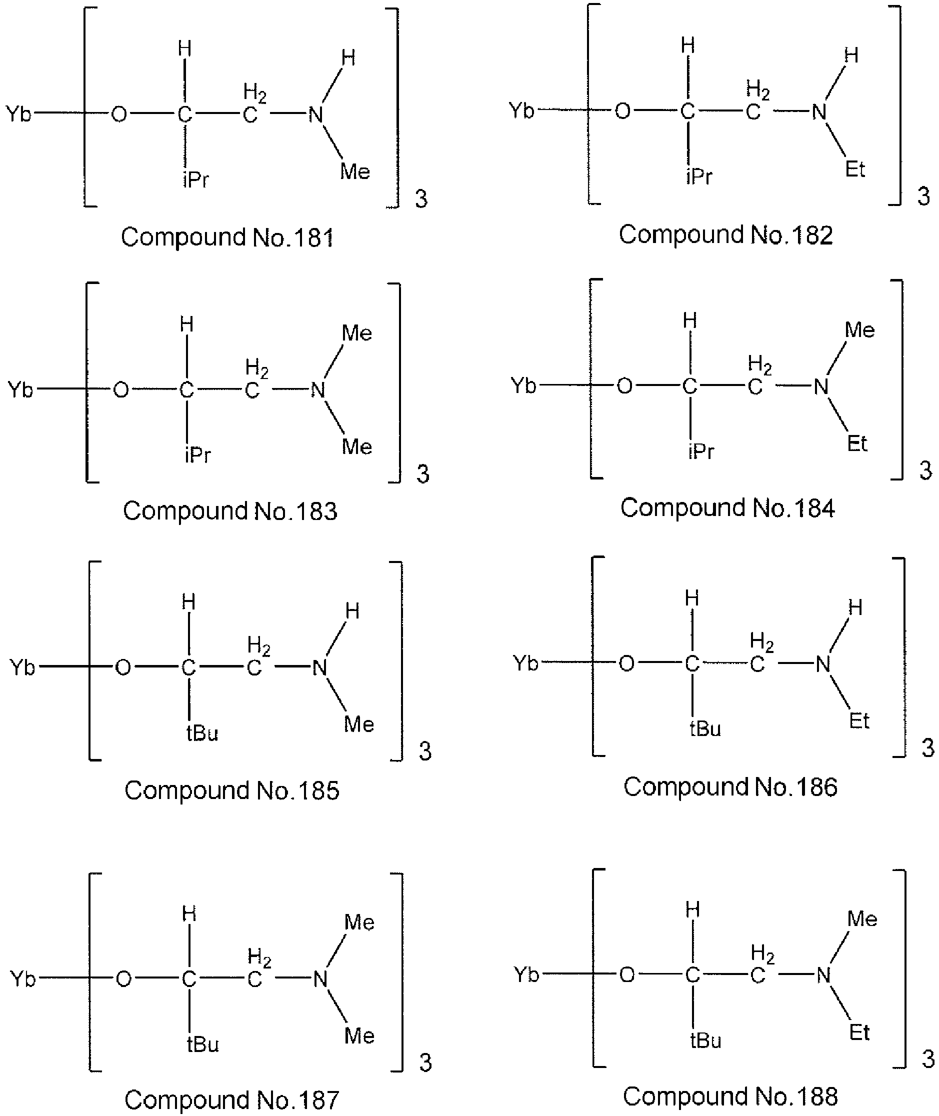

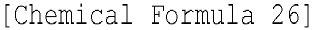

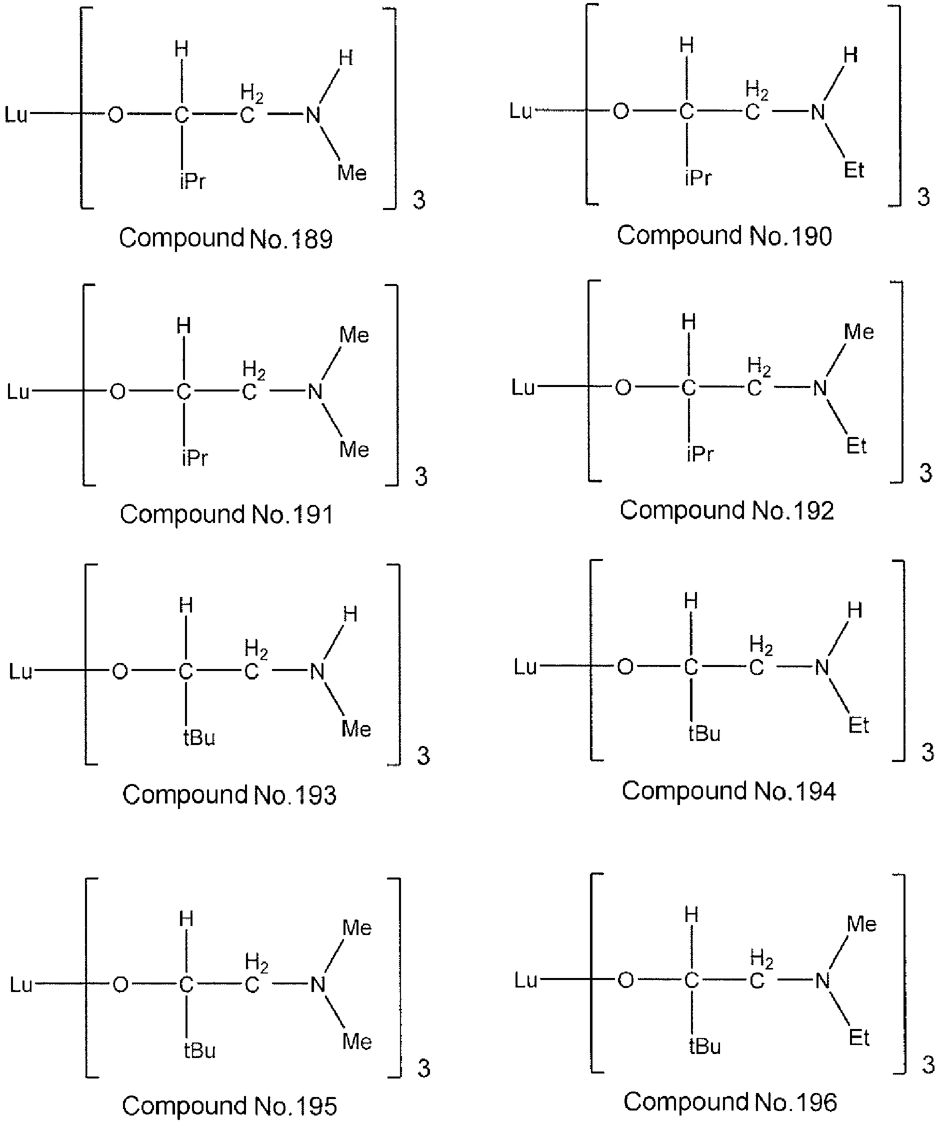

The metal alkoxide compound of the present invention is not particularly limited by the production method and can be produced by applying a known reaction. Regarding an alkoxide compound production method, a synthesis method of a generally known alkoxide compound using a corresponding alcohol can be used. For example, when a lutetium alkoxide compound is produced, a method in which, for example, inorganic salts such as lutetium halides, nitrates or their hydrates, and a corresponding alcohol compound are reacted in the presence of a base such as sodium, sodium hydride, sodium amide, sodium hydroxide, sodium methylate, ammonia, and amine; a method in which inorganic salts such as lutetium halides, nitrates or their hydrates are reacted with an alkali metal alkoxide such as a sodium alkoxide, a lithium alkoxide, and a potassium alkoxide of a corresponding alcohol compound; a method in which a low-molecular-weight alcohol alkoxide compound such as lutetium methoxide, ethoxide, isopropoxide, and butoxide and a corresponding alcohol compound are subjected to an interchange reaction, and a method in which inorganic salts such as lutetium halides and nitrates are reacted with derivatives that yield reactive intermediates to obtain reactive intermediates and then the intermediates are reacted with a corresponding alcohol compound are known. Examples of reactive intermediates include bis(dialkylamino)lutetium, bis(bis(trimethylsilyl)amino)lutetium, and lutetium amide compounds.

Here, in thin film forming raw materials of the present invention, the metal alkoxide compound of the present invention described above is used as a precursor of a thin film, and its form differs depending on a production process in which the thin film forming raw materials are applied. For example, when a thin film containing only at least one atom selected from among a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom and a lutetium atom is produced, the thin film forming raw materials of the present invention do not contain a metal compound other than the metal alkoxide compound and a semi-metal compound. On the other hand, when a thin film containing two or more metals and/or a semi-metal is produced, the thin film forming raw materials of the present invention can contain, in addition to the above metal alkoxide compound, a compound containing a desired metal and/or a compound containing a semi-metal (hereinafter also referred to as other precursors). As will be described below, the thin film forming raw materials of the present invention may further contain an organic solvent and/or a nucleophilic reagent. The thin film forming raw materials of the present invention are particularly useful as raw materials for chemical vapor deposition (hereinafter also referred to as a "raw material for CVD") because, as described above, physical properties of the metal alkoxide compound as a precursor are suitable for a CVD method and an ALD method.

When the thin film forming raw materials of the present invention are raw materials for chemical vapor deposition, their forms are appropriately selected by a method such as a transport supply method in the CVD method used or the like.

Regarding the transport supply method, a gas transport method in which a raw material for CVD is vaporized into a vapor by being heated and/or depressurized in a container in which the raw material is stored (hereinafter also simply referred to as a "raw material container"), and together with a carrier gas such as argon, nitrogen, and helium used as necessary, the vapor is introduced into a film-forming chamber in which a substrate is provided (hereinafter referred to as a "deposition reaction site") and a liquid transport method in which a raw material for CVD in a liquid or a solution state is transported to a vaporization chamber and is vaporized into a vapor by being heated and/or depressurized in the vaporization chamber, and the vapor is introduced into a film-forming chamber may be exemplified. In the case of the gas transport method, the metal alkoxide compound represented by general formula (I) can be directly used as a raw material for CVD. In the case of the liquid transport method, the metal alkoxide compound itself represented by general formula (I) or a solution in which the compound is dissolved in an organic solvent can be used as raw materials for CVD. Such a raw material for CVD may further contain other precursors, a nucleophilic reagent, and the like.

In addition, in the multi-component CVD method, a method in which components in raw materials for CVD are independently vaporized and supplied (hereinafter also referred to as a "single source method") and a method in which mixed raw materials in which multiple component raw materials are mixed in a desired composition in advance are vaporized and supplied (hereinafter also referred to as a "cocktail source method") are used. In the case of the cocktail source method, a mixture containing the metal alkoxide compound of the present invention and other precursors or a mixed solution in which the mixture is dissolved in an organic solvent can be used as raw materials for CVD. The mixture or mixed solution may further contain a nucleophilic reagent and the like. Here, when only the metal alkoxide compound of the present invention is used as a precursor and an R form and an S form are used together, a raw material for CVD including an R form and a raw material for CVD including an S form may be separately vaporized or a raw material for CVD including a mixture of an R form and an S form may be vaporized.

Regarding the organic solvent, a generally known organic solvent can be used without any particular limitation. Examples of organic solvents include acetate esters such as ethyl acetate, butyl acetate, and methoxyethyl acetate; ethers such as tetrahydrofuran, tetrahydropyran, ethylene glycol dimethyl ether, diethylene glycol dimethyl ether, triethylene glycol dimethyl ether, dibutyl ether, and dioxane; ketones such as methyl butyl ketone, methyl isobutyl ketone, ethyl butyl ketone, dipropyl ketone, diisobutyl ketone, methyl amyl ketone, cyclohexanone, and methyl cyclohexanone; hydrocarbons such as hexane, cyclohexane, methylcyclohexane, dimethylcyclohexane, ethylcyclohexane, heptane, octane, toluene, and xylene; hydrocarbons having a cyano group such as 1-cyanopropane, 1-cyanobutane, 1-cyanohexane, cyanocyclohexane, cyanobenzene, 1,3-dicyanopropane, 1,4-dicyanobutane, 1,6-dicyanohexane, 1,4-dicyanocyclohexane, and 1,4-dicyanobenzene; pyridine, and lutidine. These may be used alone or as a solvent in which two or more thereof are mixed depending on the solubility of a solute, and the relationships between an operation temperature, a boiling point, and a flash point, and the like. When such an organic solvent is used, preferably, a total amount of the precursors in a raw material for CVD, which is a solution in which the precursors are dissolved in the organic solvent, is 0.01 to 2.0 mol/liter, and particularly 0.05 to 1.0 mol/liter. When the thin film forming raw materials of the present invention do not contain a metal compound other than the metal alkoxide compound of the present invention and a semi-metal compound, a total amount of the precursors is an amount of the metal alkoxide compound of the present invention, and when the thin film forming raw materials of the present invention contain a compound containing another metal in addition to the metal alkoxide compound and/or a compound containing a semi-metal, a total amount of the precursors is a total amount of the metal alkoxide compound of the present invention and other precursors.

In addition, in the case of the multi-component CVD method, regarding other precursors to be used together with the metal alkoxide compound of the present invention, a generally known precursor used in a raw material for CVD can be used without any particular limitation.

Regarding the other precursors, compounds including one, two or more selected from the group consisting of compounds used as organic ligands such as an alcohol compound, a glycol compound, a β-diketone compound, a cyclopentadiene compound, and an organic amine compound, and silicon and a metal may be exemplified. In addition, examples of types of metal precursor include lithium, sodium, potassium, magnesium, calcium, strontium, barium, titanium, zirconium, hafnium, vanadium, niobium, tantalum, chromium, molybdenum, tungsten, manganese, iron, ruthenium, cobalt, rhodium, iridium, nickel, palladium, platinum, copper, silver, gold, zinc, aluminum, gallium, indium, germanium, tin, lead, antimony, and bismuth.

Examples of an alcohol compound used as an organic ligand for the other precursors include alkyl alcohols such as methanol, ethanol, propanol, isopropyl alcohol, butanol, sec-butyl alcohol, isobutyl alcohol, tert-butyl alcohol, pentyl alcohol, isopentyl alcohol, and tert-pentyl alcohol; ether alcohols such as 2-methoxyethanol, 2-ethoxyethanol, 2-butoxyethanol, 2-(2-methoxyethoxy)ethanol, 2-methoxy-1-methylethanol, 2-methoxy-1,1-dimethylethanol, 2-ethoxy-1,1-dimethylethanol, 2-isopropoxy-1,1-dimethylethanol, 2-butoxy-1,1-dimethylethanol, 2-(2-methoxyethoxy)-1,1-dimethylethanol, 2-propoxy-1,1-diethylethanol, 2-s-butoxy-1,1-diethylethanol, and 3-methoxy-1,1-dimethylpropanol; and dialkylamino alcohols such as dimethylaminoethanol, ethylmethylaminoethanol, diethylaminoethanol, dimethylamino-2-pentanol, ethylmethylamino-2-pentanol, dimethylamino-2-methyl-2-pentanol, ethyl methylamino-2-methyl-2-pentanol, and diethylamino-2-methyl-2-pentanol.

Examples of a glycol compound used as an organic ligand for the other precursors include 1,2-ethanediol, 1,2-propanediol, 1,3-propanediol, 2,4-hexanediol, 2,2-dimethyl-1,3-propanediol, 2,2-diethyl-1,3-propanediol, 1,3-butanediol, 2,4-butanediol, 2,2-diethyl-1,3-butanediol, 2-ethyl-2-butyl-1,3-propanediol, 2,4-pentanediol, 2-methyl-1,3-propanediol, 2-methyl-2,4-pentanediol, 2,4-hexanediol, and 2,4-dimethyl-2,4-pentanediol.

In addition, examples of β-diketone compounds include alkyl-substituted β-diketones such as acetylacetone, hexane-2,4-dione, 5-methylhexane-2,4-dione, heptane-2,4-dione, 2-methylheptane-3,5-dione, 5-methylheptane-2,4-dione, 6-methylheptane-2,4-dione, 2,2-dimethylheptane-3,5-dione, 2,6-dimethylheptane-3,5-dione, 2,2,6-trimethylheptane-3,5-dione, 2,2,6,6-tetramethylheptane-3,5-dione, octane-2,4-dione, 2,2,6-trimethyloctane-3,5-dione, 2,6-dimethyloctane-3,5-dione, 2,9-dimethylnonane-4,6-dione, 2-methyl-6-ethyldecane-3,5-dione, and 2,2-dimethyl-6-ethyldecane-3,5-dione; fluorine-substituted alkyl β-diketones such as 1,1,1-trifluoropentane-2,4-dione, 1,1,1-trifluoro-5,5-dimethylhexane-2,4-dione, 1,1,1,5,5,5-hexafluoropentane-2,4-dione, and 1,3-diperfluorohexylpropane-1,3-dione; and ether-substituted β-diketones such as 1,1,5,5-tetramethyl-1-methoxyhexane-2,4-dione, 2,2,6,6-tetramethyl-1-methoxyheptane-3,5-dione, and 2,2,6,6-tetramethyl-1-(2-methoxyethoxy)heptane-3,5-dione.

In addition, examples of cyclopentadiene compounds include cyclopentadiene, methylcyclopentadiene, ethylcyclopentadiene, propylcyclopentadiene, isopropylcyclopentadiene, butylcyclopentadiene, sec-butylcyclopentadiene, isobutylcyclopentadiene, tert-butylcyclopentadiene, dimethylcyclopentadiene, and tetramethylcyclopentadiene, and examples of organic amine compounds used as the organic ligand include methylamine, ethylamine, propylamine, isopropylamine, butylamine, sec-butylamine, tert-butylamine, isobutylamine, dimethylamine, diethylamine, dipropylamine, diisopropylamine, ethylmethylamine, propylmethylamine, and isopropylmethylamine.

The other precursors are known in the related art and methods of producing the same are known. Regarding a production method example, for example, when an alcohol compound is used as an organic ligand, an inorganic salt of a metal or its hydrates as described above is reacted with an alkali metal alkoxide of the alcohol compound, and thereby a precursor can be produced. Here, examples of inorganic salts of metals or their hydrates include metal halides and nitrates, and examples of alkali metal alkoxides include sodium alkoxides, lithium alkoxides, and potassium alkoxides.

In the case of a single source method, the other precursors are preferably compounds having a similar thermal and/or oxidative decomposition behavior to those of the metal alkoxide compound of the present invention, and in the case of a cocktail source method, a compound which has a similar thermal and/or oxidative decomposition behavior and also does not cause deterioration due to a chemical reaction or the like during mixing is preferable.

In addition, the thin film forming raw materials of the present invention may contain, as necessary, a nucleophilic reagent for imparting stability to the metal alkoxide compound of the present invention and other precursors. Examples of nucleophilic reagents include ethylene glycol ethers such as glyme, diglyme, triglyme, and tetraglyme, crown ethers such as 18-crown-6, dicyclohexyl-18-crown-6, 24-crown-8, dicyclohexyl-24-crown-8, and dibenzo-24-crown-8, polyamines such as ethylenediamine, N,N'-tetramethylethylenediamine, diethylenetriamine, triethylenetetramine, tetraethylenepentamine, pentaethylenehexamine, 1,1,4,7,7-pentamethyldiethylenetriamine, 1,1,4,7,10,10-hexamethyltriethylenetetramine, and triethoxytriethyleneamine, cyclic polyamines such as cyclam and cyclen, heterocyclic compounds such as pyridine, pyrrolidine, piperidine, morpholine, N-methylpyrrolidine, N-methylpiperidine, N-methylmorpholine, tetrahydrofuran, tetrahydropyran, 1,4-dioxane, oxazole, thiazole, and oxathiolane, β-ketoesters such as methyl acetoacetate, ethyl acetoacetate, and 2-methoxyethyl acetoacetate, and β-diketones such as acetylacetone, 2,4-hexanedione, 2,4-heptanedione, 3,5-heptanedione, and dipivaloylmethane. An amount of such a nucleophilic reagent used is preferably in a range of 0.1 mol to 10 mol and more preferably in a range of 1 to 4 mol with respect to 1 mol of a total amount of the precursors.

In the thin film forming raw materials of the present invention, an impurity metal element content, an impurity halogen content such as chlorine impurities, and organic impurity components other than components constituting the same are minimized. The impurity metal element content is preferably 100 ppb or less and more preferably 10 ppb or less for each element, and a total amount thereof is preferably 1 ppm or less and more preferably 100 ppb or less. In particular, when used as a gate insulating film, a gate film, or a barrier layer of an LSI, it is necessary to reduce the content of alkali metal elements and alkaline earth metal elements which affect electrical characteristics of the obtained thin film. The impurity halogen content is preferably 100 ppm or less, more preferably 10 ppm or less, and most preferably 1 ppm or less. A total amount of organic impurity components is preferably 500 ppm or less, more preferably 50 ppm or less, and most preferably 10 ppm or less. In addition, since water causes generation of particles in the raw material for chemical vapor deposition and generation of particles during thin film formation, in order to reduce the amount of water in each of the precursor, the organic solvent, and the nucleophilic reagent, it is preferable to remove as much water as possible in advance during use. The content of water in each of the precursor, the organic solvent, and the nucleophilic reagent is preferably 10 ppm or less and more preferably 1 ppm or less.

In addition, in the thin film forming raw materials of the present invention, in order to reduce the amount of or prevent particle contaminants in the formed thin film, it is preferable that as few particles as possible be contained. Specifically, in measurement of particles by a particle detector in a light scattering liquid in a liquid phase, the number of particles larger than 0.3 µm is preferably 100 or less in 1 mL of a liquid phase, the number of particles larger than 0.2 µm is more preferably 1,000 or less in 1 mL of a liquid phase, and the number of particles larger than 0.2 µm is most preferably 100 or less in 1 mL of a liquid phase.

The method of producing a thin film of the present invention in which a thin film is produced using the thin film forming raw materials of the present invention is a CVD method in which a vapor obtained by vaporizing the thin film forming raw materials of the present invention and a reactive gas used as necessary are introduced into a film-forming chamber in which a substrate is provided (processing atmosphere), and next, a precursor is decomposed and/or chemically reacted on the substrate and a metal-containing thin film grows and is deposited on the surface of the substrate. Regarding raw material transport supply methods, deposition methods, production conditions, production devices, and the like, generally known conditions and methods can be used without any particular limitation.

Examples of reactive gases used as necessary include oxygen, ozone, nitrogen dioxide, nitrogen monoxide, water vapor, hydrogen peroxide, formic acid, acetic acid, and acetic anhydride which have oxidizability and hydrogen which has reducibility, and examples of those for producing nitrides include organic amine compounds such as monoalkylamines, dialkylamines, trialkylamines, and alkylenediamines, hydrazine, and ammonia, and one, two or more thereof can be used. Among these, since the thin film forming raw materials of the present invention then have favorable reactivity with ozone, when one reactive gas is used, ozone is preferably used, and when a gas in which two or more thereof are mixed is used as a reactive gas, it is preferable to include at least ozone.

In addition, examples of transport supply methods include the gas transport method, liquid transport method, single source method, and cocktail source method described above.

In addition, examples of deposition methods include thermal CVD in which a thin film is deposited by reacting a raw material gas or a raw material gas and a reactive gas only by heating, plasma CVD using heat and plasma, photo CVD using heat and light, photo plasma CVD using heat, light and plasma, and ALD in which a CVD deposition reaction is divided into elementary processes and stepwise deposition is performed at the molecular level.

Examples of materials of the substrate include silicon; ceramics such as silicon nitride, titanium nitride, tantalum nitride, titanium oxide, titanium nitride, ruthenium oxide, zirconium oxide, hafnium oxide, and lanthanum oxide; glass; and metals such as metal ruthenium. Examples of shapes of the substrate include a plate shape, a spherical shape, a fibrous shape, and a scaly shape. The surface of the substrate may be flat or it may have a three-dimensional structure such as a trench structure.

In addition, the production conditions include reaction temperature (substrate temperature), reaction pressure, deposition rate, and the like. The reaction temperature is preferably 100°C or higher which is a temperature at which the compound of the present invention sufficiently reacts, and more preferably 150°C to 400°C, and particularly preferably 200°C to 350°C. In addition, the reaction pressure is preferably 10 Pa to atmospheric pressure in the case of thermal CVD or photo CVD, and is preferably 10 Pa to 2,000 Pa when a plasma is used.

In addition, the deposition rate can be controlled according to raw material supply conditions (vaporization temperature, vaporization pressure), reaction temperature, and reaction pressure. The deposition rate is preferably 0.01 nm/min to 100 nm/min and more preferably 1 nm/min to 50 nm/min because characteristics of the obtained thin film may deteriorate if the deposition rate is large and problems may occur in productivity if the deposition rate is small. In addition, in the ALD method, the number of cycles is controlled to obtain a desired film thickness.

The production conditions further include a temperature and pressure at which the thin film forming raw materials are vaporized into a vapor. A process of vaporizing the thin-film-forming raw materials into a vapor may be performed in the raw material container or in the vaporization chamber. In any case, the thin-film-forming raw materials of the present invention are preferably evaporated at 0°C to 150°C. In addition, when the thin-film-forming raw materials are vaporized into a vapor in the raw material container or the vaporization chamber, the pressure in the raw material container and the pressure in the vaporization chamber are both preferably 1 Pa to 10,000 Pa.

The method of producing a thin film of the present invention adopts an ALD method, and may include, in addition to a raw material introducing process in which thin film forming raw materials are vaporized into a vapor, and the vapor is introduced into a film-forming chamber by a transport supply method, a precursor thin film forming process in which a precursor thin film is formed on the surface of the substrate using a compound in the vapor, an exhaust process in which unreacted gas compounds are exhausted, and a metal-containing thin film forming process in which the precursor thin film is chemically reacted with a reactive gas to form a metal-containing thin film on the surface of the substrate.

Hereinafter, respective processes of the ALD method will be described in detail using a case in which a metal oxide thin film is formed as an example. First, the above raw material introducing process is performed. A preferable temperature and pressure when thin film forming raw materials are vaporized are the same as those described in the method of producing a thin film according to the CVD method. Next, the vapor introduced into the film-forming chamber is brought into contact with the surface of the substrate, and thereby a precursor thin film is formed on the surface of the substrate (precursor thin film forming process). In this case, the substrate may be heated or the film-forming chamber may be heated to apply heat. The precursor thin film formed in this process is a thin film formed from the compound of the present invention or a thin film formed by decomposition and/or reaction of a part of the compound of the present invention, and has a composition different from that of a desired metal oxide thin film. A substrate temperature when this process is performed is preferably room temperature to 500°C and more preferably 150°C to 350°C. The pressure of the system (in the film-forming chamber) in which this process is performed is preferably 1 Pa to 10,000 Pa and more preferably 10 Pa to 1,000 Pa.

Next, unreacted gas compounds and byproduct gases are exhausted from the film-forming chamber (exhaust process). The unreacted gas compounds and byproduct gases should ideally be completely exhausted from the film-forming chamber, but they are not necessarily completely exhausted. Examples of an exhaust method include a method in which the inside of the system is purged with an inert gas such as nitrogen, helium, and argon, a method of exhausting by reducing the pressure in the system, and a combination method thereof. When the pressure is reduced, the degree of pressure reduction is preferably 0.01 Pa to 300 Pa and more preferably 0.01 Pa to 100 Pa.

Next, an oxidizing gas as a reactive gas is introduced into the film-forming chamber, and a metal oxide thin film is formed from the precursor thin film obtained in the previous precursor thin film forming process according to the action of the oxidizing gas or the action of the oxidizing gas and heat (metal-oxide-containing thin film forming process). A temperature when heat is applied in this process is preferably room temperature to 500°C and more preferably 150°C to 350°C. The pressure in the system (in the film-forming chamber) in which this process is performed is preferably 1 Pa to 10,000 Pa and more preferably 10 Pa to 1,000 Pa. Since the compound of the present invention has favorable reactivity with an oxidizing gas, it is possible to obtain a metal oxide thin film having a small residual carbon content and high quality.

In the method of producing a thin film of the present invention, when the above ALD method is adopted, thin film deposition according to a series of operations including the above raw material introducing process, precursor thin film forming process, exhaust process and metal-oxide-containing thin film forming process is set as one cycle, and this cycle may be repeated a plurality of times until a thin film with a required film thickness is obtained. In this case, after one cycle is performed, in the same manner as in the exhaust process, unreacted gas compounds and reactive gases (an oxidizing gas when a metal oxide thin film is formed), and additionally preferably, byproduct gases are exhausted from the deposition reaction site and the next cycle is then performed.

In addition, in formation of a metal oxide thin film according to the ALD method, energy such as that in plasma, light, and voltage may be applied or a catalyst may be used. The time for which the energy is applied and the time for which a catalyst is used are not particularly limited, and the time may be, for example, during introduction of a compound gas in the raw material introducing process, during heating in the precursor thin film forming process or in the metal oxide containing thin film forming process, during exhausting of the system in the exhaust process, or during introduction of an oxidizing gas in the metal oxide containing thin film forming process, or may be between these processes.

In addition, in the method of producing a thin film of the present invention, after thin film deposition, in order to obtain more favorable electrical characteristics, an annealing treatment may be performed under an inert atmosphere, an oxidizing atmosphere, or reducing atmosphere, and a reflow process may be provided when step coverage is necessary. The temperature in this case is 200°C to 1,000°C and preferably 250°C to 500°C.

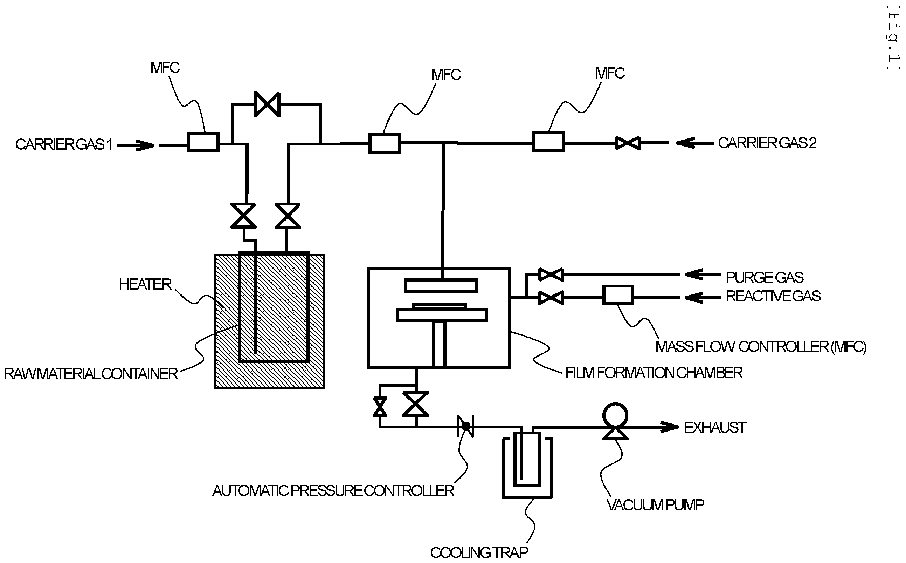

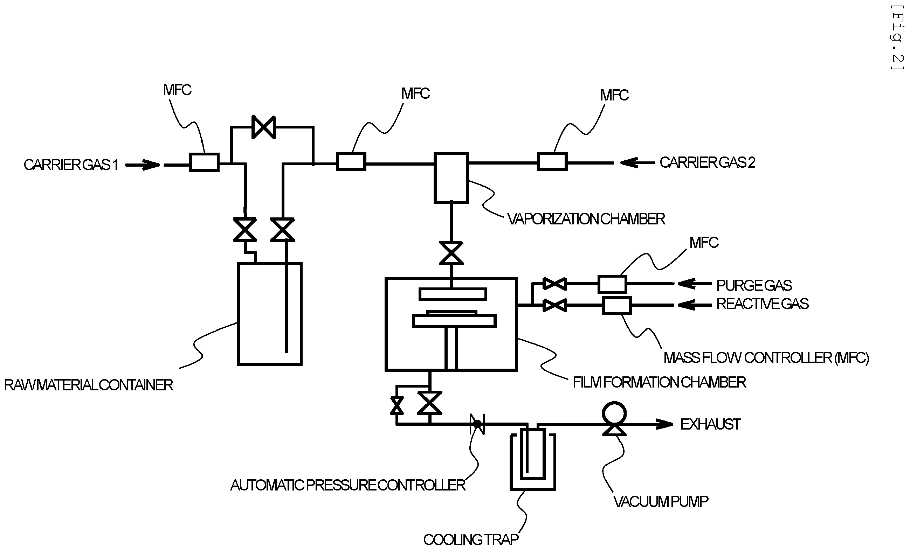

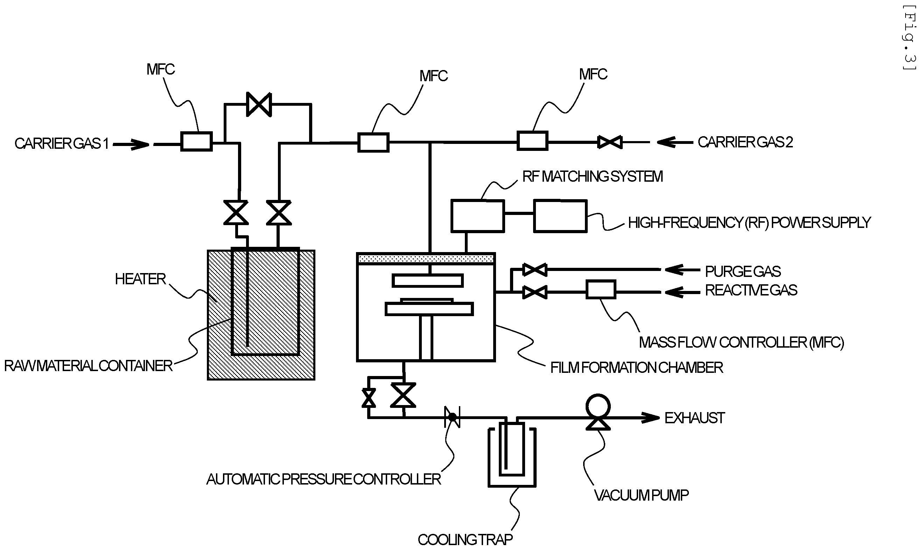

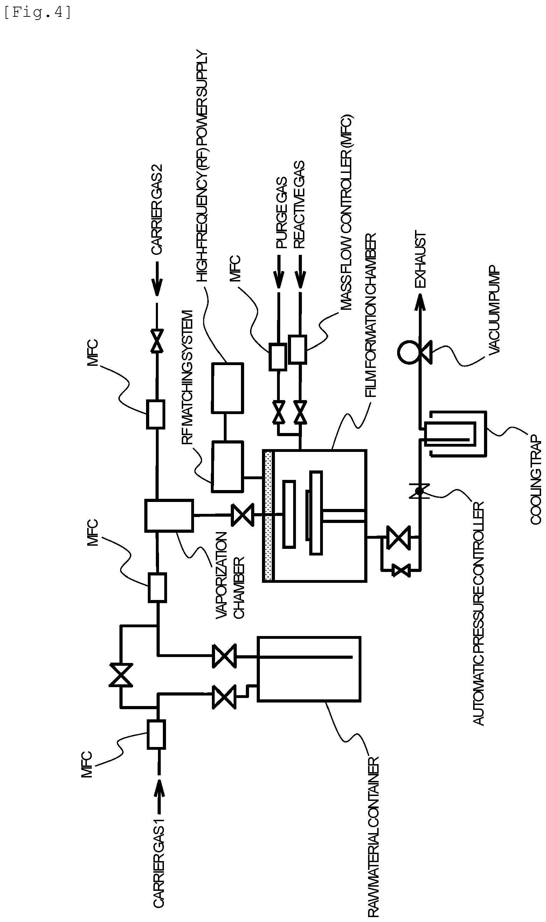

Regarding a device for producing a thin film using the thin film forming raw materials of the present invention, a known chemical vapor deposition device can be used. Specific examples of devices include a device that can perform bubbling supply of a precursor as shown in Fig. 1 and a device having a vaporization chamber as shown in Fig. 2. In addition, a device that can perform a plasma treatment on a reactive gas as shown in Fig. 3 and Fig. 4 may be exemplified. A device that can simultaneously process a plurality of wafers using a batch furnace can be used without limitation in addition to a single-wafer type device as shown in Fig. 1 to Fig. 4.

A thin film produced using the thin film forming raw materials of the present invention can be made into a thin film of a desired type such as a metal, an oxide ceramic, a nitride ceramic, and a glass by appropriately selecting other precursors, reactive gases and production conditions. It is known that thin films exhibit certain electrical characteristics, optical characteristics, and the like, and are applied in various applications. For example, an yttrium-containing thin film is used as an YBCO-based superconductor. In addition, a lanthanum-containing thin film is used as a ferroelectric PLZT. In addition, many lanthanoid atoms are used as dopants for imparting a function of improving electrical characteristics of a specific thin film and a function of improving luminosity.

### Examples

Hereinafter, the present invention will be described in more detail with reference to examples, production examples, comparative examples, and evaluation examples. However, the present invention is not limited to the following examples and the like.

### [Example 1] Synthesis of Compound No. 12

2.2 g of yttrium-tris-trimethylsilylamide and 33 g of dehydrated toluene were put into a reaction flask and sufficiently mixed. 3.4 g of 1-ethylmethylamino-3-methylbutan-2-ol was added dropwise to the obtained suspension at room temperature (20°C). After stirring at room temperature for 19 hours, the solvent was removed in an oil bath at 111°C under a reduced pressure. The generated yttrium complex (orange viscous liquid) was put into a flask, which was connected to a Kugelrohr purification device, and distillation was performed at a heating temperature of 260°C and at a pressure of 15 Pa, and thereby 1.2 g of a light yellow viscous liquid was obtained.

### (Analysis values)

(1) Atmospheric pressure TG-DTA
   Mass 50% reduction temperature: 328°C (Ar flow rate: 100 ml/min, temperature increase 10°C/min)
(2) Reduced pressure TG-DTA
   Mass 50% reduction temperature: 254°C (Ar flow rate: 50 ml/min, temperature increase 10°C/min)
(3) 1H-NMR (heavy benzene)
   0.82-1.44 ppm (10H, multiplet), 1.85-2.68 (7H, broad), 3.10-4.30 ppm (1H, broad)
(4) Elemental analysis (theoretical values)
   C: 54.6% (55.26%), H: 10.2% (10.44%), Y: 16.4% (17.04%), N: 8.9% (8.06%), O: 9.3% (9.20%)

### [Example 2] Synthesis of Compound No. 15

2.2 g of yttrium-tris-trimethylsilylamide and 20 g of dehydrated toluene were put into a reaction flask and sufficiently mixed. 1.7 g of 1-dimethylamino-3,3-dimethylbutan-2-ol was added dropwise to the obtained suspension at room temperature (20°C). After heating and stirring in an oil bath at 90°C for 8 hours, the solvent was removed in an oil bath at 110°C under a reduced pressure. The generated yttrium complex (white solid) was put into a flask, which was connected to a Kugelrohr purification device, and distillation was performed at a heating temperature of 195°C and at a pressure of 30 Pa, and thereby 1.0 g of a colorless transparent viscous liquid was obtained.

### (Analysis values)

(1) Atmospheric pressure TG-DTA
   Mass 50% reduction temperature: 308°C (Ar flow rate: 100 ml/min, temperature increase 10°C/min)
(2) Reduced pressure TG-DTA
   Mass 50% reduction temperature: 220°C (Ar flow rate: 50 ml/min, temperature increase 10°C/min)
(3) 1H-NMR (heavy benzene)
   1.00-1.25 ppm (9 H, multiplet), 1.90-2.75 (8 H, broad), 3.00-4.40 ppm (1 H, broad)
(4) Elemental analysis (theoretical values)
   C: 55.3% (55.26%), H: 10.1% (10.44%), Y: 16.5% (17.04%), N: 8.7% (8.06%), O: 9.0% (9.20%)

### [Example 3] Synthesis of Compound No. 16

2.0 g of yttrium-tris-trimethylsilylamide and 20 g of dehydrated toluene were put into a reaction flask and sufficiently mixed. 1.7 g of 1-ethylmethylamino-3,3-dimethylbutan-2-ol was added dropwise to the obtained suspension at room temperature (20°C) . After heating and stirring in an oil bath at 80°C to 90°C for 12 hours, the solvent was removed in an oil bath at 110°C under a reduced pressure. The generated yttrium complex (white solid) was put into a flask, which was connected to a Kugelrohr purification device, and distillation was performed at a heating temperature of 195°C and at a pressure of 30 Pa, and thereby 1.0 g of a colorless transparent viscous liquid was obtained.

### (Analysis values)

(1) Atmospheric pressure TG-DTA
   Mass 50% reduction temperature: 320°C (Ar flow rate: 100 ml/min, temperature increase 10°C/min)
(2) Reduced pressure TG-DTA
   Mass 50% reduction temperature: 239°C (Ar flow rate: 50 ml/min, temperature increase 10°C/min)
(3) 1H-NMR (heavy benzene)
   1.00-1.25 ppm (12 H, multiplet), 2.20-3.30 (7 H, multiplet), 3.70-4.10 ppm (1 H, broad)
(4) Elemental analysis (theoretical values)
   C: 56.2% (57.53%), H: 10.0% (10.73%), Y: 16.2% (15.77%), N: 7.7% (7.45%), O: 9.0% (8.51%)

### [Example 4] Synthesis of Compound No. 187

43.35 g of ytterbium-tris-trimethylsilylamide and 363.43 g of dehydrated toluene were put into a reaction flask and sufficiently mixed. 40.16 g of 1-dimethylamino-3,3-dimethylbutan-2-ol was added dropwise to the obtained suspension at room temperature (20°C) . After stirring at room temperature for 18 hours, the solvent was removed in an oil bath at 111°C under a reduced pressure. The generated ytterbium complex (light yellow solid) was put into a flask, which was connected to a distillation purification device, and a ribbon heater covered from a non-heated part of the oil bath to the top of a recovery flask. Distillation was performed in an oil bath at 178°C and in the ribbon heater at 162°C and at a pressure of 12 Pa, and thereby 27 g of a light yellow solid was obtained. The melting point of the obtained light yellow solid was 56°C.

### (Analysis values)

(1) Atmospheric pressure TG-DTA
   Mass 50% reduction temperature: 275°C (Ar flow rate: 100 ml/min, temperature increase 10°C/min)
(2) Reduced pressure TG-DTA
   Mass 50% reduction temperature: 193°C (Ar flow rate: 50 ml/min, temperature increase 10°C/min)
(3) Elemental analysis (theoretical values)
   C: 46.2% (47.59%), H: 9.1% (8.99%), Yb: 29.2% (28.57%), N: 6.9% (6.94%), O: 7.6% (7.92%)

### [Example 5] Synthesis of Compound No. 195

2.2 g of lutetium-tris-trimethylsilylamide and 20 g of dehydrated toluene were put into a reaction flask and sufficiently mixed. 3.2 g of 1-dimethylamino-3,3-dimethylbutan-2-ol was added dropwise to the obtained suspension at room temperature (20°C). After stirring overnight, the solvent was removed in an oil bath at 90°C under a reduced pressure. The generated lutetium complex (white viscous liquid) was put into a Kugelrohr purification device and purified. Purification was performed at a device heating temperature of 190°C and at a pressure of 12 Pa, and thereby 1.4 g of a white solid was obtained. The melting point of the obtained white solid was 127°C.

### (Analysis values)

(1) Atmospheric pressure TG-DTA
   Mass 50% reduction temperature: 282°C (Ar flow rate: 100 ml/min, temperature increase 10°C/min)
(2) Reduced pressure TG-DTA
   Mass 50% reduction temperature: 202°C (Ar flow rate: 50 ml/min, temperature increase 10°C/min)
(3) 1H-NMR (heavy benzene)
   0.91-1.31 ppm (9 H, broad), 2.02-2.69 (8 H, broad), 3.35-3.78 ppm (1 H, broad)
(4) Elemental analysis (theoretical values)
   C: 47.2% (47.44%), H: 8.4% (8.96%), Lu: 27.9% (28.79%), N: 7.2% (6.91%), O: 7.5% (7.90%)

### [Example 6] Synthesis of Compound No. 196

2.0 g of lutetium-tris-trimethylsilylamide and 28 g of dehydrated toluene were put into a reaction flask and sufficiently mixed. 3.2 g of 1-ethylmethylamino-3,3-dimethylbutan-2-ol was added dropwise to the obtained suspension at room temperature (20°C) . After stirring overnight, the solvent was removed in an oil bath at 110°C under a reduced pressure. The generated lutetium complex (white opaque viscous liquid) was put into a Kugelrohr purification device and purified. Purification was performed at a device heating temperature of 210°C and at a pressure of 11 Pa, and thereby 0.40 g of a high viscosity white liquid was obtained.

### (Analysis values)

(1) Atmospheric pressure TG-DTA
   Mass 50% reduction temperature: 305°C (Ar flow rate: 100 ml/min, temperature increase 10°C/min)
(2) Reduced pressure TG-DTA
   Mass 50% reduction temperature: 232°C (Ar flow rate: 50 ml/min, temperature increase 10°C/min)
(3) 1H-NMR (heavy benzene)
   0.78-1.27 ppm (12 H, multiplet), 2.08-3.40 ppm (7 H, broad), 3.70-4.20 ppm (1 H, broad)
(4) Elemental analysis (theoretical values)
   C: 48.7% (49.91%), H: 9.1% (9.31%), Lu: 26.7% (26.93%), N: 6.4% (6.47%), O: 7.2% (7.39%)

### [Example 7] Synthesis of Compound No. 139

2.2 g of dysprosium-tris-trimethylsilylamide and 29 g of dehydrated toluene were put into a reaction flask and sufficiently mixed. 2.0 g of 1-dimethylamino-3,3-dimethylbutan-2-ol was added dropwise to the obtained suspension at room temperature (20°C). After stirring at room temperature for 24 hours, the solvent was removed in an oil bath at 110°C under a reduced pressure. The generated dysprosium complex (light yellow solid) was put into a flask, which was connected to a Kugelrohr purification device, and distillation was performed at a heating temperature of 170°C and at a pressure of 35 Pa, and thereby 0.10 g of a white solid was obtained.

### (Analysis values)

(1) Atmospheric pressure TG-DTA
   Mass 50% reduction temperature: 294°C (Ar flow rate: 100 ml/min, temperature increase 10°C/min)
(2) Reduced pressure TG-DTA
   Mass 50% reduction temperature: 205°C (Ar flow rate: 50 ml/min, temperature increase 10°C/min)
(3) Elemental analysis (theoretical values)
   C: 48.2% (48.43%), H: 9.3% (9.14%), Dy: 27.0% (27.30%), N: 7.4% (7.06%), O: 8.2% (8.06%)

### [Example 8] Synthesis of Compound No. 147

0.55 g of holmium-tris-trimethylsilylamide and 4.7 g of dehydrated toluene were put into a reaction flask and sufficiently mixed. 0.49 g of 1-dimethylamino-3,3-dimethylbutan-2-ol was added dropwise to the obtained suspension at room temperature (20°C). After stirring at room temperature for 18 hours, the solvent was removed in an oil bath of 100°C under a reduced pressure. The generated holmium complex (yellow solid) was put into a flask, which was connected to a Kugelrohr purification device, and distillation was performed at a heating temperature of 170°C and at a pressure of 54 Pa, and thereby a white solid was obtained.

### (Analysis values)

(1) Atmospheric pressure TG-DTA
   Mass 50% reduction temperature: 306°C (Ar flow rate: 100 ml/min, temperature increase 10°C/min)
(2) Reduced pressure TG-DTA
   Mass 50% reduction temperature: 219°C (Ar flow rate: 50 ml/min, temperature increase 10°C/min)
(3) Elemental analysis (theoretical values)
   C: 48.1% (48.23%), H: 9.5% (9.11%), Ho: 27.2% (27.60%), N: 6.8% (7.03%), O: 7.7% (8.03%)

### [Evaluation Examples 1 to 8] Evaluation of physical properties of alkoxide compound

Regarding the metal alkoxide compounds Nos. 12, 15, 16, 187, 195, 196, 139, and 147 of the present invention obtained in Examples 1 to 8 and the following Comparative Compounds 1, 2, and 3, using a TG-DTA measuring device, a temperature (L) when the weight of the sample was reduced by 50 mass% due to heating under a reduced pressure atmosphere (10 torr) was checked. It was possible to determine that a compound having a low L was preferable because the vapor pressure was then high. The results are shown in Table 1.

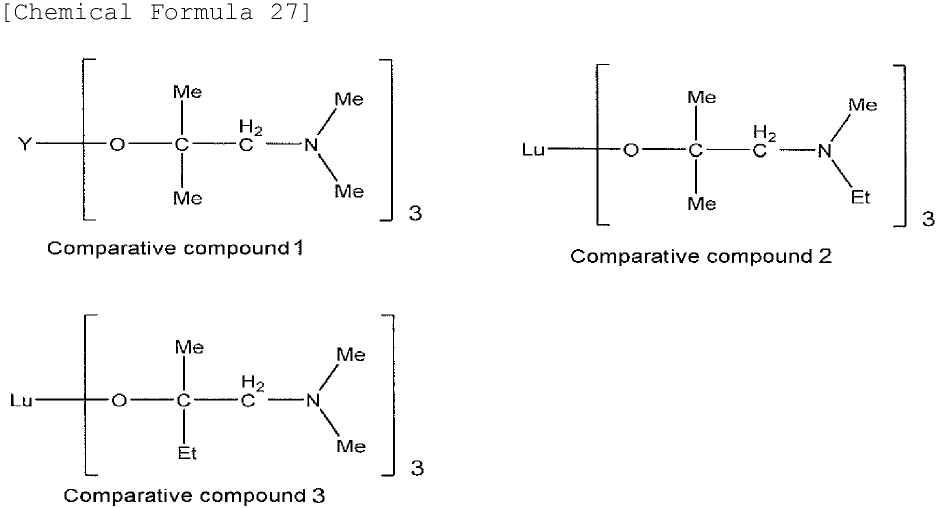

### [Table 1]

**Table 1**

| | Compound | Central metal | L |
|---|---|---|---|
| Evaluation Example 1 | Compound No. 12 | Yttrium | 250°C |
| Evaluation Example 2 | Compound No. 15 | Yttrium | 220°C |
| Evaluation Example 3 | Compound No. 16 | Yttrium | 240°C |
| Evaluation Example 4 | Compound No. 187 | Ytterbium | 190°C |
| Evaluation Example 5 | Compound No. 195 | Lutetium | 200°C |
| Evaluation Example 6 | Compound No. 196 | Lutetium | 230°C |
| Evaluation Example 7 | Compound No. 139 | Dysprosium | 210°C |
| Evaluation Example 8 | Compound No. 147 | Holmium | 220°C |
| Comparative Example 1 | Comparative Compound 1 | Yttrium | 290°C |
| Comparative Example 2 | Comparative Compound 2 | Lutetium | 270°C |
| Comparative Example 3 | Comparative Compound 3 | Lutetium | 270°C |

Based on the results in Table 1, it was found that, among metal alkoxide compounds of the present invention, Compounds Nos. 12, 15 and 16 in which a central metal was yttrium had an L that was 40°C to 70°C lower than that of Comparative Compound 1. In addition, Compound No. 187 in which a central metal was ytterbium had an L that was lower than 200°C and had a very high vapor pressure. In addition, it was found that Compounds Nos. 195 and 196 in which a central metal was lutetium had an L that was 40°C to 70°C lower than that of Comparative Compounds 2 and 3. In addition, it was found that Compound No. 139 in which a central metal was dysprosium and Compound No. 147 in which a central metal was holmium had an L that was lower than 230°C and had a very high vapor pressure. That is, it was found that, since the metal alkoxide compound of the present invention had a very high vapor pressure, it was a compound suitable as thin-film-forming raw materials for a CVD method or an ALD method.

### [Example 9] Production of yttrium oxide thin film

Compound No. 12 was used as a raw material for an atomic layer deposition method, and an yttrium oxide thin film was produced on a silicon wafer according to the ALD method under the following conditions using the device shown in Fig. 1.

When the thin film composition of the obtained thin film was checked through X-ray photoelectron spectroscopy, the obtained thin film had a composition of yttrium oxide (Y:O = 2:3), and the residual carbon content was smaller than 1.0 atom%. In addition, when the film thickness was measured according to an X-ray reflectance method and its average value was calculated, the film thickness on average was 35 nm, and the film thickness obtained for one cycle on average was 0.07 nm.

### (Conditions)

### Substrate: silicon wafer

### Reaction temperature (silicon wafer temperature): 250°C Reactive gas: H₂O

A series of processes including the following (1) to (4) was set as one cycle and repeated over 500 cycles:
(1) A raw material for an atomic layer deposition method vaporized under conditions of a raw material container temperature of 130 °C and a pressure in the raw material container of 100 Pa was introduced into a film-forming chamber and deposition was performed at a system pressure of 100 Pa for 30 seconds;
(2) Purging with argon gas was performed for 15 seconds and thereby non-deposited raw materials were removed;
(3) A reactive gas was introduced into the film-forming chamber and reacted at a system pressure of 100 Pa for 0.2 seconds; and
(4) Purging with argon gas was performed for 15 seconds and thereby unreacted first reactive gases and by-product gases were removed.

### [Example 10] Production of yttrium oxide thin film

An yttrium oxide thin film was produced under the same conditions as in Example 7 except that Compound No. 15 was used as a raw material for an atomic layer deposition method. When the thin film composition of the obtained thin film was checked through X-ray photoelectron spectroscopy, the obtained thin film had a composition of yttrium oxide (Y:O = 2:3), and the residual carbon content was smaller than 1.0 atom%. In addition, when the film thickness was measured according to an X-ray reflectance method and its average value was calculated, the film thickness on average was 40 nm, and the film thickness obtained for one cycle on average was 0.08 nm.

### [Example 11] Production of yttrium oxide thin film

An yttrium oxide thin film was produced under the same conditions as in Example 7 except that Compound No. 16 was used as a raw material for an atomic layer deposition method. When the thin film composition of the obtained thin film was checked through X-ray photoelectron spectroscopy, the obtained thin film had a composition of yttrium oxide (Y:O = 2:3), and the residual carbon content was smaller than 1.0 atom%. In addition, when the film thickness was measured according to an X-ray reflectance method and its average value was calculated, the film thickness on average was 35 nm, and the film thickness obtained for one cycle on average was 0.07 nm.

### [Comparative Example 4] Production of yttrium oxide thin film

An yttrium oxide thin film was produced under the same conditions as in Example 7 except that Comparative Compound 1 was used as a raw material for an atomic layer deposition method. When the thin film composition of the obtained thin film was checked through X-ray photoelectron spectroscopy, the obtained thin film had a composition of yttrium oxide (Y:O = 2:3), and the residual carbon content was 6.0 atom%. In addition, when the film thickness was measured according to an X-ray reflectance method and its average value was calculated, the film thickness on average was 10 nm, and the film thickness obtained for one cycle on average was 0.02 nm. Based on the results of the cross section observed through FE-SEM, the surface of the thin film was smooth.

Based on the results of Examples 9 to 11, it was found that it was possible to produce an yttrium oxide thin film having a small residual carbon content and high quality in all of the examples. On the other hand, it was found that the thin film obtained in Comparative Example 4 was an yttrium oxide thin film having a very large residual carbon content and poor quality. In addition, comparing the film thicknesses obtained for one cycle in Examples 9 to 11 and Comparative Example 4, it was found that, in Examples 9 to 11, it was possible to produce yttrium oxide with a productivity at least three times that of Comparative Example 4.

### [Example 12] Production of ytterbium oxide thin film

Compound No. 187 was used as a raw material for an atomic layer deposition method, and an ytterbium oxide thin film was produced on a silicon wafer according to the ALD method under the following conditions using the device shown in Fig. 1. When the thin film composition of the obtained thin film was checked through X-ray photoelectron spectroscopy, the obtained thin film had a composition of ytterbium oxide (Yb:O = 2:3), and the residual carbon content was smaller than 1.0 atom%. In addition, when the film thickness was measured according to an X-ray reflectance method and its average value was calculated, the film thickness on average was 40 nm, and the film thickness obtained for one cycle on average was 0.08 nm.

### (Conditions)

### Substrate: silicon wafer

### Reaction temperature (silicon wafer temperature): 250°C Reactive gas: H₂O

A series of processes including the following (1) to (4) was set as one cycle and repeated over 500 cycles:
(1) A raw material for an atomic layer deposition method vaporized under conditions of a raw material container temperature of 130°C and a pressure in the raw material container of 100 Pa was introduced into a film-forming chamber and deposition was performed at a system pressure of 100 Pa for 30 seconds;
(2) Purging with argon gas was performed for 15 seconds and thereby un-deposited raw materials were removed;
(3) A reactive gas was introduced into the film-forming chamber and reacted at a system pressure of 100 Pa for 0.2 seconds; and
(4) Purging with argon gas was performed for 15 seconds and thereby unreacted first reactive gases and by-product gases were removed.

Based on the results of Example 12, it was found that it was possible to produce an ytterbium oxide thin film having a small residual carbon content and high quality. In addition, it was found that the film thickness obtained for one cycle in Example 12 was very large at 0.08 nm and thus it was possible to produce an ytterbium oxide thin film with high productivity.

### [Example 13] Production of lutetium oxide thin film

Compound No. 195 was used as a raw material for an atomic layer deposition method, and a lutetium oxide thin film was produced on a silicon wafer according to the ALD method under the following conditions using the device shown in Fig. 1. When the thin film composition of the obtained thin film was checked through X-ray photoelectron spectroscopy, the obtained thin film had a composition of lutetium oxide (Lu:O = 2:3), and the residual carbon content was smaller than 1.0 atom%. In addition, when the film thickness was measured according to an X-ray reflectance method and its average value was calculated, the film thickness on average was 40 nm, and the film thickness obtained for one cycle on average was 0.08 nm.

### (Conditions)

### Substrate: silicon wafer

### Reaction temperature (silicon wafer temperature): 250°C Reactive gas: H₂O

A series of processes including the following (1) to (4) was set as one cycle and repeated over 500 cycles:
(1) A raw material for an atomic layer deposition method vaporized under conditions of a raw material container temperature of 130°C and a pressure in the raw material container of 100 Pa was introduced into a film-forming chamber and deposition was performed at a system pressure of 100 Pa for 30 seconds;
(2) Purging with argon gas was performed for 15 seconds and thereby un-deposited raw materials were removed;
(3) A reactive gas was introduced into the film-forming chamber and reacted at a system pressure of 100 Pa for 0.2 seconds; and
(4) Purging with argon gas was performed for 15 seconds and thereby unreacted first reactive gases and by-product gases were removed.

### [Example 14] Production of lutetium oxide thin film

A lutetium oxide thin film was produced under the same conditions as in Example 13 except that Compound No. 196 was used as a raw material for an atomic layer deposition method. When the thin film composition of the obtained thin film was checked through X-ray photoelectron spectroscopy, the obtained thin film had a composition of lutetium oxide (Lu:O = 2:3), and the residual carbon content was smaller than 1.0 atom%. In addition, when the film thickness was measured according to an X-ray reflectance method and its average value was calculated, the film thickness on average was 35 nm, and the film thickness obtained for one cycle on average was 0.07 nm.

### [Comparative Example 5] Production of lutetium oxide thin film

A lutetium oxide thin film was produced under the same conditions as in Example 13 except that Comparative Compound 2 was used as a raw material for an atomic layer deposition method. When the thin film composition of the obtained thin film was checked through X-ray photoelectron spectroscopy, the obtained thin film had a composition of lutetium oxide (Lu:O = 2:3), and the residual carbon content was 7.0 atom%. In addition, when the film thickness was measured according to an X-ray reflectance method and its average value was calculated, the film thickness on average was 10 nm, and the film thickness obtained for one cycle on average was 0.02 nm.

### [Comparative Example 6] Production of lutetium oxide thin film

A lutetium oxide thin film was produced under the same conditions as in Example 13 except that Comparative Compound 3 was used as a raw material for an atomic layer deposition method. When the thin film composition of the obtained thin film was checked through X-ray photoelectron spectroscopy, the obtained thin film had a composition of lutetium oxide (Lu:O = 2:3), and the residual carbon content was 8.0 atom%. In addition, when the film thickness was measured according to an X-ray reflectance method and its average value was calculated, the film thickness on average was 10 nm, and the film thickness obtained for one cycle on average was 0.02 nm.

Based on the results of Example 13 and Example 14, it was found that it was possible to produce a lutetium oxide thin film having a small residual carbon content and high quality in both examples. On the other hand, it was found that the thin films obtained in Comparative Example 5 and Comparative Example 6 were lutetium oxide thin films having a very large residual carbon content and poor quality. In addition, comparing film thicknesses obtained for one cycle in Example 13 and Example 14, and Comparative Example 5 and Comparative Example 6, it was found that, in Example 13 and Example 14, it was possible to produce a lutetium oxide with a productivity at least three times those in Comparative Example 5 and Comparative Example 6.

### [Example 15] Production of dysprosium oxide thin film

Compound No. 139 was used as a raw material for an atomic layer deposition method, and a dysprosium oxide thin film was produced on a silicon wafer according to the ALD method under the following conditions using the device shown in Fig. 1. When the thin film composition of the obtained thin film was checked through X-ray photoelectron spectroscopy, the obtained thin film had a composition of dysprosium oxide (Dy:O = 2:3), and the residual carbon content was smaller than 1.0 atom%. In addition, when the film thickness was measured according to an X-ray reflectance method and its average value was calculated, the film thickness on average was 300 nm, and the film thickness obtained for one cycle on average was 0.06 nm.

### (Conditions)

### Substrate: silicon wafer

### Reaction temperature (silicon wafer temperature): 250°C Reactive gas: H₂O

A series of processes including the following (1) to (4) was set as one cycle and repeated over 500 cycles:
(1) A raw material for an atomic layer deposition method vaporized under conditions of a raw material container temperature of 130°C and a pressure in the raw material container of 100 Pa was introduced into a film-forming chamber and deposition was performed at a system pressure of 100 Pa for 30 seconds;
(2) Purging with argon gas was performed for 15 seconds and thereby un-deposited raw materials were removed;
(3) A reactive gas was introduced into the film-forming chamber and reacted at a system pressure of 100 Pa for 0.2 seconds; and
(4) Purging with argon gas was performed for 15 seconds and thereby unreacted first reactive gases and by-product gases were removed.

Based on the results of Example 15, it was found that it was possible to produce a dysprosium oxide thin film having a small residual carbon content and high quality. In addition, it was found that, since the film thickness obtained for one cycle in Example 15 was very large at 0.06 nm, it was possible to produce a dysprosium oxide thin film with high productivity.

### [Example 16] Production of holmium oxide thin film

Compound No. 147 was used as a raw material for an atomic layer deposition method, and a holmium oxide thin film was produced on a silicon wafer according to the ALD method under the following conditions using the device shown in Fig. 1. When the thin film composition of the obtained thin film was checked through X-ray photoelectron spectroscopy, the obtained thin film had a composition of holmium oxide (Ho:O = 2:3), and the residual carbon content was smaller than 1.0 atom%. In addition, when the film thickness was measured according to an X-ray reflectance method and its average value was calculated, the film thickness on average was 30 nm, and the film thickness obtained for one cycle on average was 0.06 nm.

### (Conditions)

### Substrate: silicon wafer

### Reaction temperature (silicon wafer temperature): 250°C Reactive gas: H₂O

A series of processes including the following (1) to (4) was set as one cycle and repeated over 500 cycles:
(1) A raw material for an atomic layer deposition method vaporized under conditions of a raw material container temperature of 130°C and a pressure in the raw material container of 100 Pa was introduced into a film-forming chamber and deposition was performed at a system pressure of 100 Pa for 30 seconds;
(2) Purging with argon gas was performed for 15 seconds and thereby un-deposited raw materials were removed;
(3) A reactive gas was introduced into the film-forming chamber and reacted at a system pressure of 100 Pa for 0.2 seconds; and
(4) Purging with argon gas was performed for 15 seconds and thereby unreacted first reactive gases and by-product gases were removed.

Based on the results of Example 16, it was found that it was possible to produce a holmium oxide thin film having a small residual carbon content and high quality. In addition, it was found that, since the film thickness obtained for one cycle in Example 16 was very large at 0.06 nm, it was possible to produce a dysprosium oxide thin film with high productivity.

## Claims

1. A metal alkoxide compound represented by the following general formula (1): 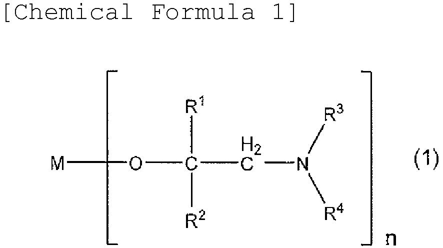 wherein the formula, R¹ represents a hydrogen atom or an alkyl group having 1 to 4 carbon atoms, R² represents an isopropyl group, a sec-butyl group, a tert-butyl group, a sec-pentyl group, a 1-ethylpropyl group or a tert-pentyl group, R³ represents a hydrogen atom or an alkyl group having 1 to 4 carbon atoms, R⁴ represents an alkyl group having 1 to 4 carbon atoms, M represents a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom or a lutetium atom, and n represents the valence of the atom represented by M; provided that when M is a lanthanum atom, R² is a sec-butyl group, a tert-butyl group, a sec-pentyl group, a 1-ethylpropyl group or a tert-pentyl group.

2. A thin film forming raw material including the metal alkoxide compound according to claim 1.

3. A method of producing a thin film containing at least one atom selected from the group consisting of a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom, and a lutetium atom on a surface of a substrate, the method comprising:
a step of introducing a vapor that contains a compound obtained by vaporizing the thin film forming raw material according to claim 2 to a treating atmosphere, and decomposing and/or chemically-reacting the compound to deposit the compound on the surface of the substrate.

## Amended claims

### Amended claims under Art. 19.1 PCT

1. [Amended] A metal alkoxide compound represented by the following general formula (1): 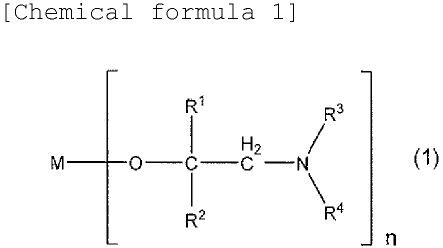 wherein R¹ represents a hydrogen atom or an alkyl group having 1 to 4 carbon atoms, R² represents an isopropyl group, a sec-butyl group, a tert-butyl group, a sec-pentyl group, a 1-ethylpropyl group or a tert-pentyl group, R³ represents a hydrogen atom or an alkyl group having 1 to 4 carbon atoms, R⁴ represents an alkyl group having 1 to 4 carbon atoms, M represents a scandium atom, an yttrium atom, a lanthanum atom, a cerium atom, a praseodymium atom, a promethium atom, a samarium atom, a europium atom, a gadolinium atom, a terbium atom, a dysprosium atom, a holmium atom, an erbium atom, a thulium atom, an ytterbium atom or a lutetium atom, and n represents the valence of the atom represented by M; here, when M is a lanthanum atom, R² is a sec-butyl group, a tert-butyl group, a sec-pentyl group, a 1-ethylpropyl group or a tert-pentyl group.

2. A thin film forming raw material including the metal alkoxide compound according to claim 1.

3. [Amended] A method of producing a thin film containing at least one atom selected from among a scandium atom, an yttrium atom, a lanthanum atom, a cerium atom, a praseodymium atom, a promethium atom, a samarium atom, a europium atom, a gadolinium atom, a terbium atom, a dysprosium atom, a holmium atom, an erbium atom, a thulium atom, an ytterbium atom, and a lutetium atom on a surface of a substrate, the method comprising:
a process in which a vapor containing a compound obtained by vaporizing the thin film forming raw material according to claim 2 is introduced into a processing atmosphere, and the compound is decomposed and/or chemically reacted and deposited on the surface of the substrate.
